# EUROPEAN PATENT APPLICATION

(11) **EP 3 560 480 A1**
(43) Date of publication of application: **30.10.2019**
(21) Application number: 17882682.2
(22) Date of filing: 20.12.2017
(51) Int. Cl.: A61K 8/64, A61K 8/34, A61K 8/36, A61K 8/55, A61K 8/86, A61K 8/92, A61Q 19/00

(54) **STICK-TYPE SOLID PREPARATION BASE FOR EXTERNAL APPLICATION TO SKIN**

(30) Priority: 21.12.2016 JP 2016248267
(71) Applicant: Nissan Chemical Corporation, Tokyo 103-6119 (JP)
(72) Inventor: SAKATA, Mizuki, Funabashi-shi Chiba 274-0052 (JP); IMOTO, Takayuki, Funabashi-shi Chiba 274-0052 (JP)
(74) Representative: Hoffmann Eitle
(86) International application number: PCT/JP2017/045739
(87) International publication number: WO 2018/117156

(57) **Abstract**

There is provided a solid base material capable of preventing "sweating" over time, which is a particularly important property required for a stick-type solid base material for external application to skin, for example, for application to cosmetic products. A stick-type solid base material for external application to skin comprising a lipidic peptide compound comprising at least one of compounds of the following Formulae (1), the similar compounds thereof or pharmaceutically usable salts of the compounds, a surfactant, water, an oily base, and a saturated or unsaturated monohydric alcohol having a carbon atom number of 8 to 30: 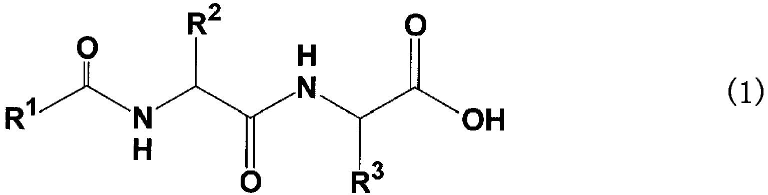 wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring.

## Description

### TECHNICAL FIELD

The present invention relates to a solid base material for external application to skin, the solid base material containing a lipidic peptide compound. Preferably, the present invention relates to a stick-type solid base material for external application to skin, the solid base material being capable of suppressing occurrence of "sweating" on the surface thereof caused by an oil separation phenomenon over time.

### BACKGROUND ART

Aqueous solid compositions have been marketed or proposed as various products for, for example, cosmetic compositions, since they provide a highly refreshing feeling upon application to, for example, the skin, and provide a dry feeling in use without leaving stickiness after use, unlike the case of oily solid compositions.

Conventionally proposed aqueous solid compositions include a solid oil-in-water makeup cosmetic composition containing water, a fatty acid soap, an oil component, and powder (Patent Document 1); and a stick-type aqueous cosmetic product containing an alkyl and/or alkenyl oligoglycoside, an oily substance, and a nonionic emulsifier (Patent Document 2).

An aqueous gel composition is an example of the aqueous solid compositions. Various compounds (e.g., a polymer gelator and a low-molecular-weight gelator) have been proposed as additives for producing such an aqueous gel. For example, a low-molecular-weight lipidic peptide gelator which has high biological safety and is expected to be applied to, for example, medical materials has recently been proposed.

### Prior Art Documents

### Patent Documents

Patent Document 1: Japanese Patent Application Publication No. H03-279319 (JP H03-279319 A)
Patent Document 2: Japanese Unexamined Patent Application Publication (Translation of PCT Application) No. 2002-516818 (JP 2002-516818 A)

### SUMMARY OF THE INVENTION

### Problems to be Solved by the Invention

An aqueous gel prepared by using the aforementioned low-molecular-weight lipidic peptide gelator has a relatively low breaking strength, which makes it difficult to apply the aqueous gel to products for applications requiring a certain level of strength, such as a stick-type solid base material for external application to skin. When such a solid base material for external application to skin is in use, the base material is expected to be frequently stored under high-temperature conditions at 50°C or higher; for example, in a vehicle in midsummer. However, for example, the aqueous gel prepared by using the aforementioned low-molecular-weight lipidic peptide gelator cannot remain in the solid state in such a high-temperature environment, and may undergo degradation of the performance or appearance of the product. Thus, an important issue is to ensure the stability of the base material against temperature (heat).

A stick-type base material for application to a cosmetic product (e.g., lipstick or foundation) contains a relatively large amount of an oily base (e.g., any oil) so as to provide both an appropriate hardness for preventing the base material from being broken or deformed upon use and an appropriate softness for allowing the base material to be smoothly spread on the skin surface. A stick-type base material for such an application may contain various pigments. However, such a stick-type base material for application to a cosmetic product may cause an oil separation phenomenon over time under some storage conditions, and liquid oil (oil droplets) may ooze on the surface of the base material as if the base material sweats. This "sweating" phenomenon may lead not only to degradation of the appearance of the stick-type base material, but also to degradation of the properties (e.g., color, odor, and the stiffness of the base material) of the cosmetic product itself.

In view of the aforementioned circumstances, an object of the present invention for solving the problems is to provide a solid base material capable of preventing "sweating" over time, which is a particularly important property required for a stick-type solid base material for external application to skin, for example, for application to cosmetic products.

### Means for Solving the Problems

The present inventors have conducted extensive studies for solving the aforementioned problems, and as a result have found that when an appropriate amount of a specific monohydric alcohol is incorporated into a stick-type solid base material for external application to skin containing, as main ingredients, a lipidic peptide compound (gelator) composed of a low-molecular-weight lipidic peptide or a pharmaceutically usable salt thereof and water, and containing an oily base so as to provide both appropriate hardness and softness, the resultant stick-type base material can suppress an oil separation phenomenon over time and thus suppress "sweating" on the surface of the base material. The present invention has been accomplished on the basis of this finding.

Accordingly, a first aspect of the present invention is a stick-type solid base material for external application to skin, the solid base material comprising:
a surfactant;
water;
a lipidic peptide compound comprising at least one of compounds of the following Formulae (1) to (3) or pharmaceutically usable salts of the compounds: 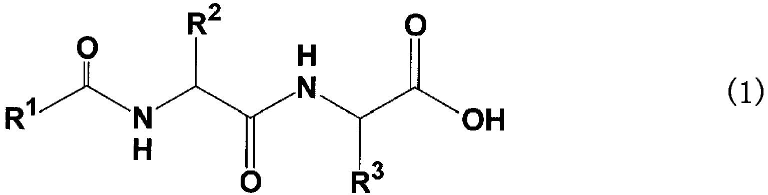 (wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring), 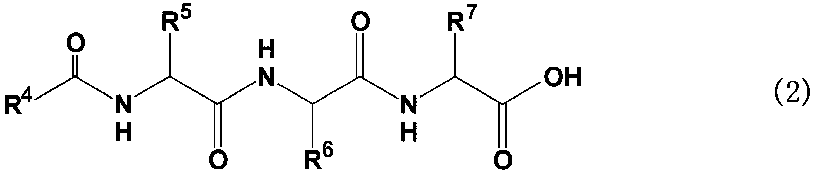 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, and at least one of R⁵ to R⁷ is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring), and 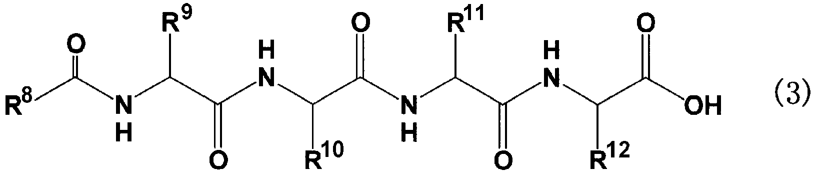 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, and at least one of R⁹ to R¹² is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring);
a 1,2-alkanediol or a 1,3-alkanediol;
at least one fatty acid;
an oily base; and
at least one saturated or unsaturated monohydric alcohol having a carbon atom number of 8 to 30.

A second aspect of the present invention is the stick-type solid base material for external application to skin according to the first aspect, wherein the monohydric alcohol is one or more compounds selected from the group consisting of cetanol, stearyl alcohol, and behenyl alcohol.

A third aspect of the present invention is the stick-type solid base material for external application to skin according to the first or second aspect, wherein the oily base is contained in an amount of 5 to 25% by mass relative to the total mass of the stick-type solid base material for external application to skin.

A fourth aspect of the present invention is the stick-type solid base material for external application to skin according to any one of the first to third aspects, wherein the surfactant is one or more compounds selected from the group consisting of an ethylene glycol alkyl ether, a phospholipid, a polyglycerin fatty acid ester, and a polyoxyethylene polyoxypropylene alkyl ether.

A fifth aspect of the present invention is the stick-type solid base material for external application to skin according to any one of the first to fourth aspects, wherein the fatty acid is stearic acid.

A sixth aspect of the present invention is the stick-type solid base material for external application to skin according to any one of the first to fifth aspects, wherein the solid base material further comprises a pigment.

### Effects of the Invention

The present invention can provide a stick-type solid base material for external application to skin, the solid base material being capable of suppressing occurrence of an oil separation phenomenon on the surface of the base material over time, which is also referred to as "sweating."

In particular, the stick-type solid base material for external application to skin of the present invention contains cetanol, stearyl alcohol, or behenyl alcohol as the monohydric alcohol. Thus, even when the oily base is contained in an amount of 25% by mass relative to the total mass of the solid base material, the aforementioned "sweating" can be suppressed.

The lipidic peptide compound contained in the stick-type solid base material for external application to skin of the present invention is a highly safe artificial low-molecular-weight compound that is composed of a lipid and a peptide only. The lipidic peptide compound enables an aqueous gel to be formed without use of, for example, a crosslinking agent, which is required for formation of a conventionally proposed synthetic polymer gel. Thus, the resultant stick-type solid base material for external application to skin does not pose a problem in terms of remaining of unreacted matter, such as unreacted crosslinking agent.

The ingredients contained, as additives, in the stick-type solid base material for external application to skin of the present invention are general-purpose additives for foods, cosmetic products, and pharmaceutical products.

Thus, the stick-type solid base material for external application to skin of the present invention has high biological safety, and is very useful for the aforementioned applications, particularly from the viewpoint of safety required in, for example, pharmaceutical products and cosmetic products.

Furthermore, the stick-type solid base material for external application to skin of the present invention is very useful as a stick-type base material for pharmaceutical products and cosmetic products, since the solid base material is expected to provide a highly refreshing feeling upon application to, for example, human skin, undergo neither breakage nor deformation, and provide good spreadability.

### BRIEF DESCRIPTION OF THE DRAWINGS

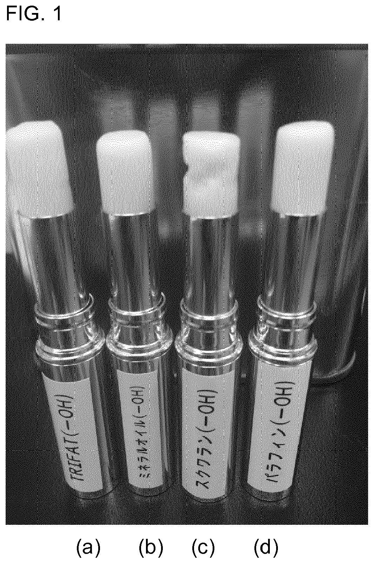

FIG. 1 shows the appearances of the surfaces of stick-type solid base materials for external application to skin (without incorporation of at least one saturated or unsaturated monohydric alcohol having a carbon atom number of 8 to 30) of Comparative Example after storage at 50°C for one week, wherein oily bases used are (a) coconut oil (TRIFAT C-24), (b) mineral oil, (c) squalane oil, and (d) liquid paraffin.

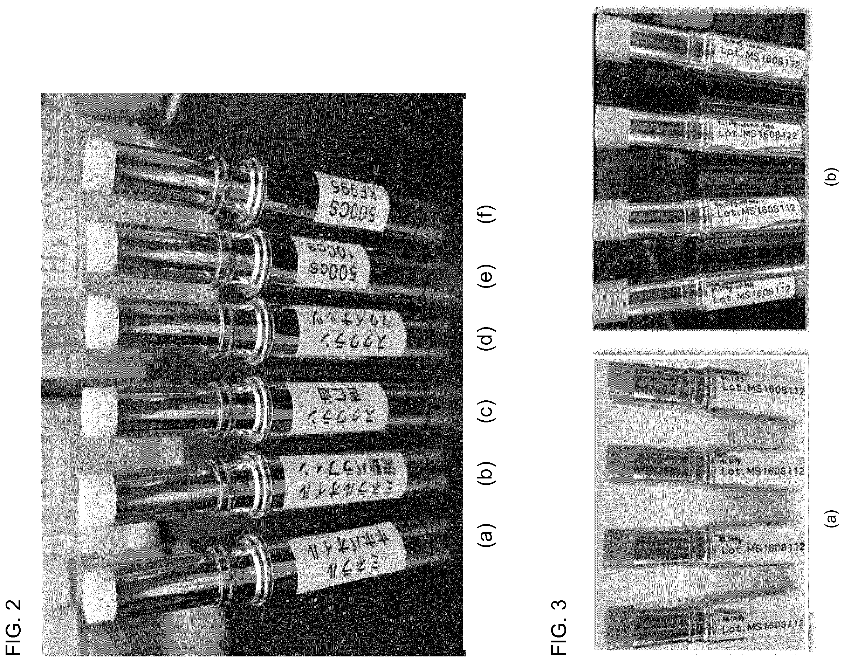

FIG. 2 shows the appearances of the surfaces of stick-type solid base materials for external application to skin (incorporation of a mixture of two oily bases) of Example 2 after storage at 50°C for 10 days, wherein oily bases used are (a) mineral oil + jojoba oil, (b) mineral oil + liquid paraffin, (c) squalane oil + apricot kernel oil, (d) squalane oil + kukui nut oil, (e) KF96A-500cs + KF96A-100cs, and (f) KF96A-500cs + KF995.
FIG. 3 shows the appearances of four samples of stick-type foundation produced in Example 3. FIG. 3(a) shows the appearances immediately after production, and FIG. 3(b) shows the appearances after storage at 50°C for four weeks.

### MODES FOR CARRYING OUT THE INVENTION

The present invention relates to a stick-type solid base material for external application to skin, the solid base material comprising a surfactant, water, a lipidic peptide compound comprising at least one of compounds of the following Formulae (1) to (3) or pharmaceutically usable salts of the compounds, a 1,2-alkanediol or 1,3-alkanediol, a fatty acid, an oily base, at least one saturated or unsaturated monohydric alcohol having a carbon atom number of 8 to 30, and optionally other additives.

The ingredients will be described below.

### [Lipidic Peptide Compound]

The lipidic peptide compound usable in the stick-type solid base material for external application to skin of the present invention is any of compounds of the following Formulae (1) to (3) (lipidic peptides) or pharmaceutically usable salts of the compounds (low-molecular-weight compounds each having a lipidic moiety as a hydrophobic moiety and a peptide moiety as a hydrophilic moiety).

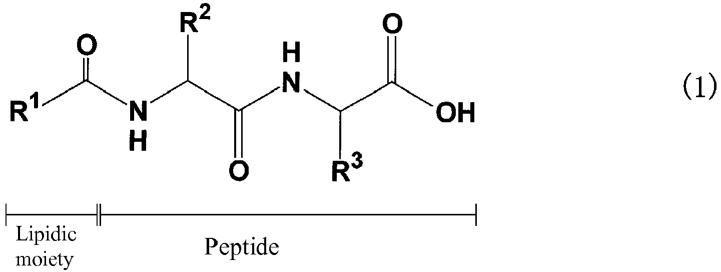

In Formula (1), R¹ is a C₉₋₂₃ aliphatic group. Preferably, R¹ is a linear aliphatic group having a carbon atom number of 11 to 23 and optionally having zero to two unsaturated bonds.

Specific examples of the lipidic moiety (acyl group) composed of R¹ and the adjacent carbonyl group include lauroyl group, dodecylcarbonyl group, myristoyl group, tetradecylcarbonyl group, palmitoyl group, margaroyl group, oleoyl group, elaidoyl group, linoleoyl group, stearoyl group, vaccenoyl group, octadecylcarbonyl group, arachidoyl group, eicosylcarbonyl group, behenoyl group, elkanoyl group, docosylcarbonyl group, lignoceroyl group, and nervonoyl group. Particularly preferred examples include lauroyl group, myristoyl group, palmitoyl group, margaroyl group, stearoyl group, oleoyl group, elaidoyl group, and behenoyl group.

In Formula (1), R² included in the peptide moiety is a hydrogen atom or a C₁₋₄ alkyl group optionally having a C₁ or C₂ branched chain.

The C₁₋₄ alkyl group optionally having a C₁ or C₂ branched chain refers to an alkyl group having a C₁₋₄ main chain and optionally having a C₁ or C₂ branched chain. Specific examples of the alkyl group include methyl group, ethyl group, n-propyl group, i-propyl group, n-butyl group, i-butyl group, sec-butyl group, and tert-butyl group.

R² is preferably a hydrogen atom or a C₁₋₃ alkyl group optionally having a C₁ branched chain, more preferably a hydrogen atom.

The C₁₋₃ alkyl group optionally having a C₁ branched chain refers to an alkyl group having a C₁₋₃ main chain and optionally having a C₁ branched chain. Specific examples of the alkyl group include methyl group, ethyl group, n-propyl group, i-propyl group, i-butyl group, and sec-butyl group. Preferred is methyl group, i-propyl group, i-butyl group, or sec-butyl group.

In Formula (1), R³ is a -(CH₂)ₙ-X group. In the -(CH₂)ₙ-X group, n is a number of 1 to 4, and X is an amino group, a guanidino group, a -CONH₂ group, or a 5-membered ring group or 6-membered ring group optionally having one to three nitrogen atoms or a fused heterocyclic group composed of the 5-membered ring and the 6-membered ring.

In the -(CH₂)ₙ-X group represented by R₃, X is preferably amino group, guanidino group, carbamoyl group (-CONH₂ group), pyrrole group, imidazole group, pyrazole group, or indole group, more preferably imidazole group. In the -(CH₂)ₙ-X group, n is preferably 1 or 2, more preferably 1.

Thus, the -(CH₂)ₙ- group is preferably aminomethyl group, 2-aminoethyl group, 3-aminopropyl group, 4-aminobutyl group, carbamoylmethyl group, 2-carbamoylethyl group, 3-carbamoylbutyl group, 2-guanidinoethyl group, 3-guanidinobutyl group, pyrrolemethyl group, 4-imidazolemethyl group, pyrazolemethyl group, or 3-indolemethyl group, more preferably 4-aminobutyl group, carbamoylmethyl group, 2-carbamoylethyl group, 3-guanidinobutyl group, 4-imidazolemethyl group, or 3-indolemethyl group, still more preferably 4-imidazolemethyl group.

Particularly suitable lipidic peptide compounds of Formula (1) are the following compounds each being formed of a lipidic moiety and a peptide moiety (amino acid assembly), wherein the amino acid abbreviations are alanine (Ala), asparagine (Asn), glutamine (Gln), glycine (Gly), histidine (His), isoleucine (Ile), leucine (Leu), lysine

(Lys), tryptophan (Trp), and valine (Val). Specific examples of the compounds include lauroyl-Gly-His, lauroyl-Gly-Gln, lauroyl-Gly-Asn, lauroyl-Gly-Trp, lauroyl-Gly-Lys, lauroyl-Ala-His, lauroyl-Ala-Gln, lauroyl-Ala-Asn, lauroyl-Ala-Trp, and lauroyl-Ala-Lys; myristoyl-Gly-His, myristoyl-Gly-Gln, myristoyl-Gly-Asn, myristoyl-Gly-Trp, myristoyl-Gly-Lys, myristoyl-Ala-His, myristoyl-Ala-Gln, myristoyl-Ala-Asn, myristoyl-Ala-Trp, and myristoyl-Ala-Lys; palmitoyl-Gly-His, palmitoyl-Gly-Gln, palmitoyl-Gly-Asn, palmitoyl-Gly-Trp, palmitoyl-Gly-Lys, palmitoyl-Ala-His, palmitoyl-Ala-Gln, palmitoyl-Ala-Asn, palmitoyl-Ala-Trp, and palmitoyl-Ala-Lys; and stearoyl-Gly-His, stearoyl-Gly-Gln, stearoyl-Gly-Asn, stearoyl-Gly-Trp, stearoyl-Gly-Lys, stearoyl-Ala-His, stearoyl-Ala-Gln, stearoyl-Ala-Asn, stearoyl-Ala-Trp, and stearoyl-Ala-Lys.

Most preferred are lauroyl-Gly-His, lauroyl-Ala-His, myristoyl-Gly-His, myristoyl-Ala-His; palmitoyl-Gly-His, palmitoyl-Ala-His; stearoyl-Gly-His, and stearoyl-Ala-His. 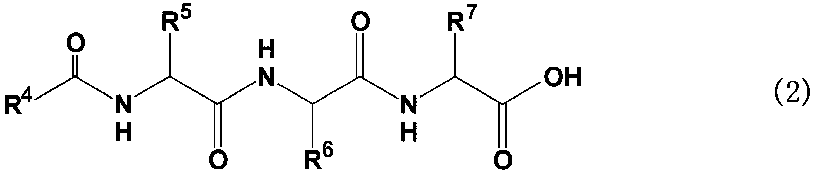
In Formula (2), R⁴ is a C₉₋₂₃ aliphatic group. Preferred specific examples of R⁴ include the same groups as those defined by R¹ above.
In Formula (2), R⁵ to R⁷ are each independently a hydrogen atom, a C₁₋₄ alkyl group optionally having a C₁ or C₂ branched chain, or a -(CH₂)ₙ-X group, and at least one of R⁵ to R⁷ is a -(CH₂)ₙ-X group. In the -(CH₂)ₙ-X group, n is a number of 1 to 4, and X is an amino group, a guanidino group, a -CONH₂ group, or a 5-membered ring group or 6-membered ring group optionally having one to three nitrogen atoms or a fused heterocyclic group composed of the 5-membered ring and the 6-membered ring. Preferred specific examples of R⁵ to R⁷ include the same groups as those defined by R² and R³ above.

Suitable lipidic peptide compounds of Formula (2) are the following compounds each being formed of a lipidic moiety and a peptide moiety (amino acid assembly). Specific examples of the compounds include lauroyl-Gly-Gly-His, lauroyl-Gly-Gly-Gln, lauroyl-Gly-Gly-Asn, lauroyl-Gly-Gly-Trp, lauroyl-Gly-Gly-Lys, lauroyl-Gly-Ala-His, lauroyl-Gly-Ala-Gln, lauroyl-Gly-Ala-Asn, lauroyl-Gly-Ala-Trp, lauroyl-Gly-Ala-Lys, lauroyl-Ala-Gly-His, lauroyl-Ala-Gly-Gln, lauroyl-Ala-Gly-Asn, lauroyl-Ala-Gly-Trp, lauroyl-Ala-Gly-Lys, lauroyl-Gly-His-Gly, lauroyl-His-Gly-Gly, myristoyl-Gly-Gly-His, myristoyl-Gly-Gly-Gln, myristoyl-Gly-Gly-Asn, myristoyl-Gly-Gly-Trp, myristoyl-Gly-Gly-Lys, myristoyl-Gly-Ala-His, myristoyl-Gly-Ala-Gln, myristoyl-Gly-Ala-Asn, myristoyl-Gly-Ala-Trp, myristoyl-Gly-Ala-Lys, myristoyl-Ala-Gly-His, myristoyl-Ala-Gly-Gln, myristoyl-Ala-Gly-Asn, myristoyl-Ala-Gly-Trp, myristoyl-Ala-Gly-Lys, myristoyl-Gly-His-Gly, myristoyl-His-Gly-Gly, palmitoyl-Gly-Gly-His, palmitoyl-Gly-Gly-Gln, palmitoyl-Gly-Gly-Asn, palmitoyl-Gly-Gly-Trp, palmitoyl-Gly-Gly-Lys, palmitoyl-Gly-Ala-His, palmitoyl-Gly-Ala-Gln, palmitoyl-Gly-Ala-Asn, palmitoyl-Gly-Ala-Trp, palmitoyl-Gly-Ala-Lys, palmitoyl-Ala-Gly-His, palmitoyl-Ala-Gly-Gln, palmitoyl-Ala-Gly-Asn, palmitoyl-Ala-Gly-Trp, palmitoyl-Ala-Gly-Lys, palmitoyl-Gly-His-Gly, palmitoyl-His-Gly-Gly, stearoyl-Gly-Gly-His, stearoyl-Gly-Gly-Gln, stearoyl-Gly-Gly-Asn, stearoyl-Gly-Gly-Trp, stearoyl-Gly-Gly-Lys, stearoyl-Gly-Ala-His, stearoyl-Gly-Ala-Gln, stearoyl-Gly-Ala-Asn, stearoyl-Gly-Ala-Trp, stearoyl-Gly-Ala-Lys, stearoyl-Ala-Gly-His, stearoyl-Ala-Gly-Gln, stearoyl-Ala-Gly-Asn, stearoyl-Ala-Gly-Trp, stearoyl-Ala-Gly-Lys, stearoyl-Gly-His-Gly, and stearoyl-His-Gly-Gly.

Of these, most preferred are lauroyl-Gly-Gly-His, myristoyl-Gly-Gly-His, palmitoyl-Gly-Gly-His, palmitoyl-Gly-His-Gly, palmitoyl-His-Gly-Gly, and stearoyl-Gly-Gly- His. 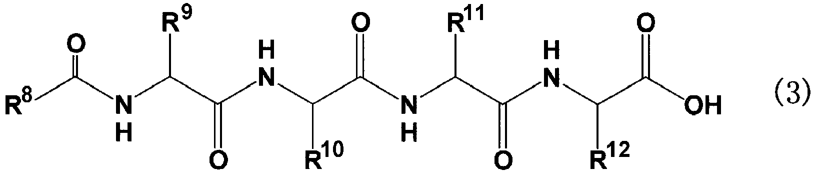
In Formula (3), R⁸ is a C₉₋₂₃ aliphatic group. Preferred specific examples of R⁸ include the same groups as those defined by R¹ above.
In Formula (3), R⁹ to R¹² are each independently a hydrogen atom, a C₁₋₄ alkyl group optionally having a C₁ or C₂ branched chain, or a -(CH₂)ₙ-X group, and at least one of R⁹ to R¹² is a -(CH₂)ₙ-X group. In the -(CH₂)ₙ-X group, n is a number of 1 to 4, and X is an amino group, a guanidino group, a -CONH₂ group, or a 5-membered ring group or 6-membered ring group optionally having one to three nitrogen atoms or a fused heterocyclic group composed of the 5-membered ring and the 6-membered ring. Preferred specific examples of R⁹ to R¹² include the same groups as those defined by R² and R³ above.

Thus, particularly suitable examples of the lipidic peptide compound of Formula (3) include lauroyl-Gly-Gly-Gly-His, myristoyl-Gly-Gly-Gly-His, palmitoyl-Gly-Gly-Gly-His, palmitoyl-Gly-Gly-His-Gly, palmitoyl-Gly-His-Gly-Gly, palmitoyl-His-Gly-Gly-Gly, and stearoyl-Gly-Gly-Gly-His.

In the present invention, the amount of the lipidic peptide compound contained in the stick-type solid base material for external application to skin is, for example, 1 to 20% by mass, preferably 1 to 10% by mass, more preferably 3 to 7% by mass, relative to the total mass of the solid base material.

The lipidic peptide compound used in the present invention is composed of at least one of compounds (lipidic peptides) of Formulae (1) to (3) or pharmaceutically usable salts of the compounds. These compounds may be used alone or in combination of two or more species as a hydrogelator.

### [Surfactant]

The surfactant usable in the stick-type solid base material for external application to skin of the present invention is preferably a compound having a hydrophilic moiety and a hydrophobic moiety in the molecule wherein the hydrophilic moiety has a betaine structure (hereinafter the compound may also be referred to as a "betaine compound"), an ethylene glycol alkyl ether, a polyglycerin fatty acid ester, or a polyoxyethylene polyoxypropylene alkyl ether.

Examples of the aforementioned betaine compound include betaine compounds known as amphoteric surfactants, for example, N-alkyl-N,N-dimethylamino acid betaines, such as lauryl dimethylaminoacetic acid betaine (lauryl betaine); fatty acid amidoalkyl-N,N-dimethylamino acid betaines, such as cocamidopropyl betaine and lauramidopropyl betaine; imidazoline betaines, such as sodium cocoamphoacetate and sodium lauroamphoacetate; alkyl sulfobetaines, such as lauryl hydroxysulfobetaine and alkyl dimethyltaurine; betaine sulfates, such as alkyl dimethylaminoethanol sulfate ester; and betaine phosphates, such as alkyl dimethylaminoethanol phosphate ester.

Other examples of the betaine compound include glycerophospholipids, such as phosphatidylcholine, phosphatidylethanolamine, phosphatidylserine, phosphatidylinositol, phosphatidylglycerol, diphosphatidylglycerol (cardiolipin), and phosphatidic acid; lysoglycerophospholipids, such as lysophosphatidylcholine (lysolecithin), lysophosphatidylethanolamine, lysophosphatidylserine, lysophosphatidylinositol, lysophosphatidylglycerol, and lysophosphatidic acid; sphingophospholipids, such as sphingomyelin; and hydrogenated additives thereof. These phospholipids may be derived from animals and plants, such as soybean and egg yolk, or may be chemically or enzymatically synthesized.

Among the above-exemplified betaine compounds, preferred are lauryl dimethylaminoacetic acid betaine, lauramidopropyl betaine, lauryl hydroxysulfobetaine, stearyl betaine, lysophosphatidylcholine (lysolecithin), lysophosphatidylethanolamine, lysophosphatidylserine, lysophosphatidylinositol, lysophosphatidylglycerol, and lysophosphatidic acid, and more preferred are lysophosphatidylcholine (lysolecithin).

Examples of the ethylene glycol alkyl ether include polyoxyethylene alkyl ethers, polyoxyethylene lauryl ethers, polyoxyethylene palmitoyl ethers, and polyoxyethylene stearyl ethers. The ethylene glycol alkyl ether may be a commercially available product. Examples of the usable commercial product include those from the EMULGEN (registered trademark) series and the EMANON (registered trademark) series available from Kao Corporation; for example, EMULGEN 102KG, EMULGEN 103, EMULGEN 104P, EMULGEN 105, EMULGEN 106, EMULGEN 108, EMULGEN 109P, EMULGEN 120, EMULGEN 123P, EMULGEN 130K, EMULGEN 147, EMULGEN 150, EMULGEN 210P, EMULGEN 220, EMULGEN 306P, EMULGEN 320P, EMULGEN 350, EMULGEN 404, EMULGEN 408, EMULGEN 409PV, EMULGEN 420, EMULGEN 430, EMULGEN 705, EMULGEN 707, EMULGEN 709, EMULGEN 1108, EMULGEN 1118S-70, EMULGEN 1135S-70, EMULGEN 1150S-60, EMULGEN 4085, EMULGEN 2020G-HA, EMULGEN 2025G, EMANON 1112, EMANON 3199V, EMANON 3299V, EMANON 3299RV, and EMANON 4110. More preferred examples include EMULGEN 103, EMULGEN 104P, EMULGEN 105, EMULGEN 106, EMULGEN 108, EMULGEN 109P, EMULGEN 210P, EMULGEN 306P, EMULGEN 320P, EMULGEN 404, EMULGEN 408, EMULGEN 409PV, EMULGEN 420, EMULGEN 705, EMULGEN 707, EMULGEN 709, EMULGEN 1108, EMULGEN 2020G-HA, EMANON 1112, and EMANON 4110 available from Kao Corporation. Still more preferred examples include EMULGEN 104P, EMULGEN 105, EMULGEN 106, EMULGEN 108, EMULGEN 210P, EMULGEN 306P, EMULGEN 408, EMULGEN 409PV, EMULGEN 705, EMULGEN 707, EMULGEN 709, EMULGEN 1108, EMULGEN 2020G-HA, EMANON 1112, and EMANON 4110 available from Kao Corporation. In addition to these examples, the ethylene glycol alkyl ether may be appropriately selected from the NIKKOL (registered trademark) series available from Nikko Chemicals Co., Ltd. Examples thereof include NIKKOL BT-5, NIKKOL BT-7, NIKKOL BT-9, NIKKOL BT-12, NIKKOL BL-2, NIKKOL BL-4.2, and NIKKOL BL-9EX. Most preferred is NIKKOL BL-4.2.

Examples of the polyglycerin fatty acid ester include glycerin fatty acid partial esters, such as glyceryl stearate, glyceryl isostearate, glyceryl palmitate, glyceryl myristate, glyceryl oleate, glyceryl cocoate, glycerin mono-cottonseed oil fatty acid esters, glycerin monoerucate, glycerin sesquioleate, glycerin α,α'-oleate pyroglutamate, and glycerin monostearate malate; polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate (decaglyceryl diisostearate), polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate.

Examples of the polyoxyethylene polyoxypropylene alkyl ether include EMULGEN (registered trademark) LS-106, EMULGEN LS-110, EMULGEN LS-114, and EMULGEN MS-110 available from Kao Corporation; and NIKKOL (registered trademark) PBC-31, NIKKOL PBC-33, NIKKOL PBC-34, NIKKOL PBC-41, NIKKOL PBC-44, NIKKOL PBN-4612, NIKKOL PBN-4620, and NIKKOL PBN-4630 available from Nikko Chemicals Co., Ltd. More preferred are EMULGEN LS-106, EMULGEN LS-110, EMULGEN LS-114, and EMULGEN MS-110. Still more preferred are EMULGEN LS-106, EMULGEN LS-110, and EMULGEN MS-110.

The surfactant usable in the present invention is preferably a surfactant having an HLB (hydrophile-lipophile balance) value of 8 to 20, more preferably a surfactant having an HLB value of 8 to 14.

Examples of such surfactants include sorbitan isostearate, steareth-8, beheneth-10, laureth-4, laureth-5, ceteth-7, oleth-8, PEG-8 glyceryl isostearate, choleth-10, PEG-10BG isostearate, PEG-30 glyceryl triisostearate, PEG-30 glyceryl triisostearate, PEG-30 glyceryl trioleate, PEG-30 trimethylolpropane triisostearate, PEG-30 hydrogenated castor oil laurate, PEG-30 hydrogenated castor oil PCA isostearate, octyldodeceth-10, PEG-12 dilaurate, sorbeth-40 tetraoleate, polyglyceryl-10 diisostearate (decaglyceryl diisostearate), PEG-20 glyceryl diisostearate, PEG-8 isostearate, PEG-10 glyceryl isostearate, PEG-60 hydrogenated castor oil triisostearate, PPG-2-deceth-7, oleth-10, hydrogenated dimer dilinoleth-20, sorbitan cocoate, isosteareth-10, steareth-11, PEG-30 trimethylolpropane trimyristate, PEG-40 hydrogenated castor oil isostearate, PEG-40 hydrogenated castor oil isostearate, PEG-40 hydrogenated castor oil PCA isostearate, laureth-7, isoceteth-10, ceteth-10, PEG-10 isostearate, PEG-10 stearate, PEG-10 oleate, PEG-10 glyceryl stearate, oleth-12, decyltetradeceth-15, choleth-15, PEG-16 dilaurate, PEG-30 hydrogenated castor oil, PEG-40 glyceryl triisostearate, PEG-40 glyceryl trioleate, PEG-40 trimethylolpropane triisostearate, PEG-40 hydrogenated castor oil laurate, and PEG-12 laurate.

In the present invention, the amount of the surfactant contained in the stick-type solid base material for external application to skin is, for example, 0.5 to 20% by mass, preferably 0.5 to 10% by mass, more preferably 0.5 to 5% by mass, relative to the total mass of the solid base material.

The surfactant used in the present invention is at least one of the aforementioned group of surfactants, and these surfactants may be used alone or in combination of two or more species.

### [1,2-Alkanediol or 1,3-Alkanediol]

The 1,2-alkanediol or 1,3-alkanediol used in the stick-type solid base material for external application to skin of the present invention has a function of enhancing the solubility of the lipidic peptide compound.

Specific examples of the 1,2-alkanediol include 1,2-pentanediol, 1,2-hexanediol, 1,2-octanediol, and 1,2-decanediol. Preferred are 1,2-pentanediol, 1,2-hexanediol, and 1,2-octanediol. More preferred is 1,2-pentanediol or 1,2-hexanediol. The 1,2-alkanediol used in the present invention is at least one of the aforementioned group of 1,2-alkanediols.

Specific examples of the 1,3-alkanediol include 1,3-propanediol, 2-methyl-1,3-propanediol, 1,3-butanediol, 3-methyl-1,3-butanediol, 1,3-pentanediol, 1,3-hexanediol, 2-ethyl-1,3-hexanediol, 2-ethyl-1,3-octanediol, and 1,3-decanediol. Preferred are 1,3-pentanediol, 1,3-hexanediol, 2-ethyl-1,3-hexanediol, and 2-ethyl-1,3-octanediol. More preferred are 2-ethyl-1,3-hexanediol and 2-ethyl-1,3-octanediol. The 1,3-alkanediol used in the present invention is at least one of the aforementioned group of 1,3-alkanediols.

These 1,2-alkanediols or 1,3-alkanediols may be used alone or in combination of two or more species.

In the present invention, the amount of the 1,2-alkanediol or 1,3-alkanediol contained in the stick-type solid base material for external application to skin is, for example, 0.5 to 20% by mass, preferably 1 to 10% by mass, more preferably 1 to 5% by mass, relative to the total mass of the solid base material.

### [Fatty Acid]

The fatty acid used in the stick-type solid base material for external application to skin of the present invention is preferably at least one selected from the group consisting of saturated and unsaturated fatty acids each having a carbon atom number of 10 to 20 and salts of these fatty acids. Examples of the fatty acid include capric acid, undecanoic acid, lauric acid, tridecanoic acid, myristic acid, pentadecanoic acid, palmitic acid, margaric acid, and stearic acid. More preferred are capric acid, lauric acid, myristic acid, palmitic acid, and stearic acid, and particularly preferred is stearic acid.

In the present invention, the amount of the fatty acid contained in the stick-type solid base material for external application to skin is, for example, 0.1 to 2.0% by mass, preferably 0.2 to 1.0% by mass, relative to the total mass of the solid base material.

The fatty acid used in the present invention is at least one of the aforementioned group of fatty acids, and these fatty acids may be used alone or in combination of two or more species.

### [Oily Base]

No particular limitation is imposed on the oily base used in the stick-type solid base material for external application to skin of the present invention. Examples of the usable oily base include higher (polyhydric) alcohols, such as oleyl alcohol, jojoba alcohol, chimyl alcohol, selachyl alcohol, batyl alcohol, hexyldecanol, isostearyl alcohol, 2-octyldodecanol, and dimer diols; aralkyl alcohols and derivatives thereof, such as benzyl alcohol; isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, long-chain branched fatty acids, dimer acids, and hydrogenated dimer acids; hydrocarbons, such as liquid paraffin (mineral oil), heavy liquid isoparaffin, light liquid isoparaffin, α-olefin oligomers, polyisobutene, hydrogenated polyisobutene, polybutene, squalane, olive-derived squalane, squalene, vaseline, and solid paraffin; waxes, such as candelilla wax, carnauba wax, rice wax, Japan wax, beeswax, montan wax, ozokerite, ceresin, paraffin wax, microcrystalline wax, petrolatum, Fischer-Tropsch wax, polyethylene wax, and ethylene-propylene copolymers; vegetable oils and fats, such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, hydrogenated jojoba oil, grape seed oil, apricot oil (apricot kernel oil), and camellia oil; animal oils and fats, such as beef tallow, milk fat, horse fat, egg-yolk oil, mink oil, and turtle oil; animal waxes, such as spermaceti, lanolin, and orange roughy oil; lanolins, such as liquid lanolin, reduced lanolin, adsorption-purified lanolin, acetylated lanolin, acetylated liquid lanolin, hydroxylated lanolin, polyoxyethylene lanolin, lanolin fatty acids, hard lanolin fatty acids, lanolin alcohol, acetylated lanolin alcohol, and acetylated (cetyl/lanolyl) ester; sterols, such as cholesterol, dihydrocholesterol, lanosterol, dihydrolanosterol, phytosterol, and cholic acid; sapogenins; saponins; sterol esters, such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroylsarcosinate, cholesteryl 12-hydroxystearate, cholesteryl macadamiate, phytosteryl macadamiate, phytosteryl isostearate, soft lanolin fatty acid cholesteryl esters, hard lanolin fatty acid cholesteryl esters, long-chain branched fatty acid cholesteryl esters, and long-chain α-hydroxy fatty acid cholesteryl esters; lipidic complexes, such as phospholipid-cholesterol complexes and phospholipid-phytosterol complexes; monohydric alcohol esters of carboxylic acids, such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isotridecyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters, such as cetyl lactate, diisostearyl malate, and hydrogenated castor oil monoisostearate; polyhydric alcohol esters of fatty acids, such as glyceryl trioctanoate (glyceryl tri-2-ethylhexanoate), glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl ester, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl oligoester, glycol distearate (ethylene glycol distearate), 3-methyl-1,5-pentanediol dineopentanoate, and 2,4-diethyl-1,5-pentanediol dineopentanoate; dimer acid or dimer diol derivatives, such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate, and hydroxyalkyl dimer dilinoleyl ether; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamide (cocamide methyl MEA); silicones, such as dimethicone (dimethylpolysiloxane), highly polymerized dimethicone (highly polymerized dimethylpolysiloxane), cyclomethicone (cyclic dimethylsiloxane, decamethylcyclopentasiloxane (which may also be referred to simply as "cyclopentasiloxane")), phenyl trimethicone, diphenyl dimethicone, phenyl dimethicone, stearoxypropyldimethylamine, (aminoethylaminopropyl methicone/dimethicone) copolymers, dimethiconol, dimethiconol crosspolymers, silicone resin, silicone rubber, amino-modified silicones such as aminopropyl dimethicone and amodimethicone, cation-modified silicones, polyether-modified silicones such as dimethicone copolyols, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine oils, such as perfluorodecane, perfluorooctane, and perfluoropolyether. These oils are preferably exemplified.

In the present invention, the amount of the oily base contained in the stick-type solid base material for external application to skin is, for example, 1 to 50% by mass, preferably 5 to 50% by mass, more preferably 5 to 20% by mass, particularly preferably 10 to 20% by mass, relative to the total mass of the solid base material.

The oily base used in the present invention is at least one of the aforementioned group of oily bases, and these oily bases may be used alone or in combination of two or more species.

### [At Least One Saturated or Unsaturated Monohydric Alcohol Having a Carbon Atom Number of 8 to 30]

The stick-type solid base material for external application to skin of the present invention contains at least one saturated or unsaturated monohydric alcohol having a carbon atom number of 8 to 30. Thus, the solid base material exhibits improved stability; specifically, the solid base material can suppress an oil separation phenomenon on the surface of the base material, which is referred to as "sweating." The at least one saturated or unsaturated monohydric alcohol having a carbon atom number of 8 to 30 is also referred to simply as a "higher alcohol" herein.

Many raw materials of these higher alcohols, including derivatives thereof, are commercially available or synthesized. The stick-type solid base material for external application to skin of the present invention may contain preferably at least one saturated or unsaturated monohydric alcohol with an alkyl group having a carbon atom number of 8 to 30, more preferably a saturated monohydric alcohol with an alkyl group having a carbon atom number of 12 to 22. In particular, cetanol (carbon atom number of 16), stearyl alcohol (carbon atom number of 18), and behenyl alcohol (carbon atom number of 22) are preferably used, from the viewpoints of their high general versatility and high effect of suppressing the aforementioned "sweating."

In the present invention, the amount of the higher alcohol contained in the solid base material for external application to skin is, for example, 0.1 to 10% by mass, preferably 0.25 to 5% by mass, more preferably 0.5 to 3% by mass, relative to the total mass of the solid base material.

The higher alcohol used in the present invention is at least one of the aforementioned group of higher alcohols, and these higher alcohols may be used alone or in combination of two or more species.

The stick-type solid base material for external application to skin of the present invention may further contain a pigment. Examples of the usable pigment include, but are not particularly limited to, inorganic white pigments, such as titanium dioxide and zinc oxide; inorganic red pigments, such as iron oxide (red iron oxide) and iron titanate; inorganic brown pigments, such as γ-iron oxide; inorganic yellow pigments, such as yellow iron oxide and ocher; inorganic black pigments, such as black iron oxide and low-order titanium oxide; inorganic violet pigments, such as mango violet and cobalt violet; inorganic green pigments, such as chromium oxide, chromium hydroxide, and cobalt titanate; inorganic blue pigments, such as ultramarine and Prussian blue; pearl pigments, such as titanium oxide-coated mica, titanium oxide-coated bismuth oxychloride, titanium oxide-coated talc, colored titanium oxide-coated mica, bismuth oxychloride, and argentine; extender pigments, such as talc, sericite, mica, kaolin, calcium carbonate, magnesium carbonate, silicic anhydride, barium sulfate, and aluminum hydroxide; metal powder pigments, such as aluminum powder, copper powder, and gold; surface-treated inorganic and metal powder pigments; organic pigments, such as zirconium, barium, or aluminum lake; and surface-treated organic pigments.

In the present invention, when a pigment is contained in the stick-type solid base material for external application to skin, the amount of the pigment contained is, for example, 1 to 50% by mass, preferably 5 to 50% by mass, more preferably 6 to 20% by mass, for example, 10 to 20% by mass, relative to the total mass of the solid base material.

The pigment used in the present invention is at least one of the aforementioned group of pigments, and these pigments may be used alone or in combination of two or more species.

### [Organic Acid]

The stick-type solid base material for external application to skin of the present invention may further contain an organic acid.

Examples of the organic acid include ascorbic acid, citric acid, lactic acid, glycolic acid, succinic acid, acetic acid, malic acid, tartaric acid, and fumaric acid. Of these, preferred are ascorbic acid, citric acid, and lactic acid, and particularly preferred are ascorbic acid and citric acid.

In the present invention, the amount of the organic acid contained in the stick-type solid base material for external application to skin is, for example, 1 to 20% by mass, preferably 1 to 10% by mass, relative to the total mass of the solid base material.

### [Polyhydric Alcohol]

The stick-type solid base material for external application to skin of the present invention may further contain a polyhydric alcohol. The polyhydric alcohol is a polyhydric alcohol different from the above-exemplified 1,2-alkanediol or 1,3-alkanediol, and specific examples of the polyhydric alcohol include glycerin, propylene glycol, and polyethylene glycol. The incorporation of the polyhydric alcohol can improve the temporal stability of the stick-type solid base material for external application to skin. Polyethylene glycol having an average molecular weight of, for example, 1,000 to 4,000 can be suitably used as the polyhydric alcohol.

In the present invention, the amount of the polyhydric alcohol contained in the stick-type solid base material for external application to skin may be, for example, 1 to 80% by mass, preferably 1 to 60% by mass, relative to the total mass of the solid base material.

### [Other Additives]

The stick-type solid base material for external application to skin of the present invention may optionally contain additives generally usable as additives for cosmetic products, quasi-drugs, and pharmaceutical products. Examples of additive ingredients such as physiologically active substances and functional substances contained in external preparations for skin (e.g., cosmetic products, quasi-drugs, or pharmaceutical products) include humectants, texture improvers, surfactants other than those described above, polymers, thickeners/gelators, solvents, antioxidants, reducing agents, oxidizers, preservatives, antimicrobial agents, antiseptics, chelating agents, pH adjusters, acids, alkalis, powders, inorganic salts, ultraviolet absorbers, whitening agents, vitamins and derivatives thereof, hair growth-promoting agents, blood circulation promoters, stimulants, hormones, anti-wrinkle agents, anti-aging agents, firming agents, cooling agents, warming agents, wound-healing promoters, abirritants, analgesics, cell activators, plant/animal/microbial extracts, antipruritics, exfoliates/keratolytic agents, antiperspirants, algefacients, astringents, enzymes, nucleic acids, perfumes, colors, coloring agents, dyes, antiphlogistics, anti-inflammatory agents, anti-asthmatic agents, anti-chronic obstructive pulmonary disease agents, anti-allergic agents, immunomodulators, anti-infective agents, and antifungal agents.

The amount of such an additive contained in the stick-type solid base material for external application to skin may vary depending on the type of the additive. The amount of the additive is, for example, about 0.1 to 20% by mass or about 0.5 to 10% by mass, relative to the total mass of the solid base material.

Preferred examples of humectants and texture improvers include polyols and polymers thereof, such as glycerin, trimethylolpropane, pentaerythritol, hexylene glycol, diglycerin, polyglycerin, diethylene glycol, dipropylene glycol, polypropylene glycol, and ethylene glycol-propylene glycol copolymers; glycol alkyl ethers, such as diethylene glycol monoethyl ether (ethoxydiglycol), ethylene glycol monoethyl ether, ethylene glycol monobutyl ether, and diethylene glycol dibutyl ether; water-soluble esters, such as polyglyceryl-10 (eicosanedioate/tetradecanedioate) and polyglyceryl-10 tetradecanedioate; sugar alcohols, such as sorbitol, xylitol, erythritol, mannitol, and maltitol; saccharides and derivatives thereof, such as glucose, fructose, galactose, mannose, threose, xylose, arabinose, fucose, ribose, deoxyribose, maltose, trehalose, lactose, raffinose, gluconic acid, glucuronic acid, cyclodextrins (α-, β-, and γ-cyclodextrins, and modified cyclodextrins such as maltosyl cyclodextrin and hydroxyalkyl cyclodextrin), β-glucan, chitin, chitosan, heparin and derivatives thereof, pectin, arabinogalactan, dextrin, dextran, glycogen, ethyl glucoside, and glucosylethyl methacrylate polymer or copolymer; hyaluronic acid and sodium hyaluronate; sodium chondroitin sulfate; mucoitin sulfate, charonin sulfate, keratosulfate, and dermatan sulfate; Tremella fuciformis extract and Tremella fuciformis polysaccharides; fucoidan; tuberose polysaccharides or naturally occurring polysaccharides; organic acids such as citric acid, tartaric acid, and lactic acid, and salts thereof; urea and derivatives thereof; 2-pyrrolidone-5-carboxylic acid and salts thereof, such as sodium salt; amino acids, such as betaine (trimethylglycine), proline, hydroxyproline, arginine, lysine, serine, glycine, alanine, phenylalanine, tyrosine, β-alanine, threonine, glutamic acid, glutamine, asparagine, aspartic acid, cysteine, cysteine, methionine, leucine, isoleucine, valine, tryptophan, histidine, and taurine, and salts thereof; protein peptides and derivatives thereof, such as collagen, fish-derived collagen, atelocollagen, gelatin, elastin, peptides derived from degraded collagen, hydrolyzed collagen, hydroxypropylammonium chloride hydrolyzed collagen, peptides derived from degraded elastin, peptides derived from degraded keratin, hydrolyzed keratin, peptides derived from degraded conchiolin, hydrolyzed conchiolin, peptides derived from degraded silk protein, hydrolyzed silk, sodium lauroyl hydrolyzed silk, peptides derived from degraded soy protein, peptides derived from degraded wheat protein, hydrolyzed wheat protein, peptides derived from degraded casein, and acylated peptides; acylated peptides, such as palmitoyl oligopeptide, palmitoyl pentapeptide, and palmitoyl tetrapeptide; silylated peptides; lactic acid bacteria culture, yeast extracts, eggshell membrane protein, bovine submaxillary mucin, hypotaurine, sesame lignan glycosides, glutathione, albumin, and whey; choline chloride and phosphorylcholine; animal and plant extract ingredients, such as placenta extract, elastin, collagen, aloe extract, Hamamelis virginiana water, Luffa cylindrica water, Chamomilla recutita extract, licorice extract, comfrey extract, silk extract, Rosa roxburghii extract, Achillea millefolium extract, Eucalyptus globulus extract, and melilot extract; and ceramides, such as natural ceramides (types 1, 2, 3, 4, 5, and 6), hydroxyceramide, pseudoceramide, sphingoglycolipid, ceramide-containing extract, and glucosylceramide-containing extract.

Preferred examples of surfactants include anionic surfactants, nonionic surfactants, cationic surfactants, amphoteric surfactants, and polymer surfactants. Preferred examples of these surfactants are as follows. Preferred examples of anionic surfactants include fatty acid salts, such as potassium laurate and potassium myristate; alkyl sulfates, such as sodium lauryl sulfate, triethanolamine lauryl sulfate, and ammonium lauryl sulfate; polyoxyethylene alkyl sulfates, such as sodium laureth sulfate and triethanolamine laureth sulfate; acyl N-methylamino acid salts, such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate, and sodium lauroyl glutamate methyl alaninate; acyl amino acid salts, such as sodium cocoyl glutamate, triethanolamine cocoyl glutamate, sodium lauroyl glutamate, sodium myristoyl glutamate, sodium stearoyl glutamate, ditriethanolamine palmitoyl aspartate, and triethanolamine cocoyl alaninate; polyoxyethylene alkyl ether acetates, such as sodium laureth acetate; succinates, such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates, such as sodium hydrogenated coconut oil fatty acid glycerin sulfates; alkylbenzene polyoxyethylene sulfates; olefin sulfonates, such as sodium α-olefin sulfonates; alkyl sulfosuccinates, such as disodium lauryl sulfosuccinate and sodium dioctyl sulfosuccinate; alkyl ether sulfosuccinates, such as disodium laureth sulfosuccinate, sodium monolauroyl monoethanolamide polyoxyethylene sulfosuccinate, and sodium lauryl polypropylene glycol sulfosuccinate; alkylbenzene sulfonates, such as sodium tetradecylbenzene sulfonate and triethanolamine tetradecylbenzene sulfonate; alkyl naphthalene sulfonates; alkane sulfonates; α-sulfofatty acid methyl ester salts; acyl isethionates; alkyl glycidyl ether sulfonates; alkyl sulfoacetates; alkyl ether phosphates, such as sodium laureth phosphate, sodium dilaureth phosphate, sodium trilaureth phosphate, and sodium monooreth phosphate; alkyl phosphates, such as potassium lauryl phosphate; sodium caseinate; alkyl aryl ether phosphates; fatty acid amide ether phosphates; phospholipids, such as phosphatidylglycerol, phosphatidylinositol, and phosphatidic acid; and silicone anionic surfactants, such as carboxylic acid-modified silicones, phosphoric acid-modified silicones, and sulfuric acid-modified silicones. Preferred examples of nonionic surfactants include polyoxyethylene alkyl ethers with various numbers of polyoxyethylene units, such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyldodecyl ethers); polyoxyethylene alkyl phenyl ethers; castor oil derivatives and hydrogenated castor oil derivatives, such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester, and polyoxyethylene hydrogenated castor oil maleate; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers, such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols, such as PPG-9 diglyceryl; glycerin fatty acid partial esters, such as glyceryl stearate, glyceryl isostearate, glyceryl palmitate, glyceryl myristate, glyceryl oleate, glyceryl coconut oil fatty acid esters, glycerin mono-cottonseed oil fatty acid esters, glycerin monoerucate, glycerin sesquioleate, glycerin α,α'-oleate pyroglutamate, and glycerin monostearate malate; polyglycerin fatty acid esters, such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono-fatty acid esters, such as ethylene glycol monostearate; propylene glycol mono-fatty acid esters, such as propylene glycol monostearate; pentaerythritol fatty acid partial esters; sorbitol fatty acid partial esters; maltitol fatty acid partial esters; maltitol ethers; sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; sugar derivative partial esters, such as sucrose fatty acid esters, methyl glucoside fatty acid esters, and trehalose undecylenoate; alkyl glucosides, such as caprylyl glucoside; alkyl polyglycosides; lanolin alcohol; reduced lanolin; polyoxyethylene fatty acid mono- and di-esters, such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters, such as polyoxyethylene monooleates, for example, polyoxyethylene glycerin monostearate, polyoxyethylene glycerin monoisostearate, and polyoxyethylene glycerin triisostearate; polyoxyethylene sorbitan fatty acid esters, such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters, such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene-modified animal and vegetable fats and oils, such as polyoxyethylene sorbitol beeswax; alkyl glyceryl ethers, such as isostearyl glyceryl ether, chimyl alcohol, selachyl alcohol, and batyl alcohol; polyhydric alcohol alkyl ethers; polyoxyethylene alkylamines; tetrapolyoxyethylene/tetrapolyoxypropylene-ethylenediamine condensates; natural surfactants, such as saponin and sophorolipid; polyoxyethylene fatty acid amides; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamides (cocamide methyl MEA); alkyl dimethylamine oxides, such as lauramine oxide, cocamine oxide, stearamine oxide, and behenamine oxide; alkyl ethoxydimethylamine oxides; polyoxyethylene alkyl mercaptans; and silicone nonionic surfactants, for example, polyether-modified silicones such as dimethicone copolyols, polysiloxane-oxyalkylene copolymers, polyglycerin-modified silicones, and sugar-modified silicones. Preferred examples of cationic surfactants include alkyl trimethylammonium chlorides, such as behentrimonium chloride, steartrimonium chloride, cetrimonium chloride, and lauryltrimonium chloride; alkyl trimethylammonium bromides, such as steartrimonium bromide; dialkyl dimethylammonium chlorides, such as distearyldimonium chloride and dicocodimonium chloride; fatty acid amide amines, such as stearamidopropyl dimethylamine and stearamidoethyl diethylamine, and salts thereof; alkyl ether amines, such as stearoxypropyldimethylamine and salts or quaternary salts thereof; fatty acid amide quaternary ammonium salts, such as long-chain branched fatty acid (12 to 31) aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidium salts; alkyl ether amine ammonium salts; alkyl trialkylene glycol ammonium salts; benzalkonium salts; benzethonium salts; pyridinium salts, such as cetylpyridinium chloride; imidazolinium salts; alkyl isoquinolinium salts; dialkyl morpholinium salts; polyamine fatty acid derivatives; and silicone cationic surfactants, such as amino-modified silicones such as aminopropyl dimethicone and amodimethicone, cation-modified silicones, cation-modified and polyether-modified silicones, and amino-modified and polyether-modified silicones. Preferred examples of amphoteric surfactants include N-alkyl-N,N-dimethylamino acid betaines, such as lauryl betaine (lauryl dimethylaminoacetic acid betaine); fatty acid amidoalkyl-N,N-dimethylamino acid betaines, such as cocamidopropyl betaine and lauramidopropyl betaine; imidazoline betaines, such as sodium cocoamphoacetate and sodium lauroamphoacetate; alkyl sulfobetaines, such as alkyl dimethyltaurine; betaine sulfates, such as alkyl dimethylaminoethanol sulfate; betaine phosphates, such as alkyl dimethylaminoethanol phosphates; phospholipids, such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid, and hydroxylated lecithin; and silicone amphoteric surfactants. Preferred examples of polymer surfactants include polyvinyl alcohol, sodium alginate, starch derivatives, tragacanth gum, and acrylic acid-alkyl methacrylate copolymers; and various silicone surfactants.

Preferred examples of polymers, thickeners, and gelators include guar gum, locust bean gum, quince seed, carrageenan, galactan, gum arabic, tara gum, tamarind, furcellaran, karaya gum, Abelmoschus manihot, cara gum, tragacanth gum, pectin, pectic acid and salts thereof, such as sodium salt, alginic acid and salts thereof, such as sodium salt, and mannan; starches, such as rice starch, corn starch, potato starch, and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; celluloses and derivatives thereof, such as methyl cellulose, ethyl cellulose, hydroxyethyl cellulose, hydroxypropyl cellulose, hydroxypropyl methylcellulose, carboxymethyl cellulose and salts thereof, such as sodium salt, methylhydroxypropyl cellulose, sodium cellulose sulfate, dialkyldimethylammonium cellulose sulfate, crystalline cellulose, and cellulose powder; starch derivatives, such as soluble starch, starch polymers such as carboxymethyl starch, methylhydroxypropyl starch, and methyl starch, starch hydroxypropyltrimonium chloride, and aluminum corn starch octenylsuccinate; alginic acid derivatives, such as sodium alginate and propylene glycol alginate; polyvinylpyrrolidone (PVP), polyvinyl alcohol (PVA), vinylpyrrolidone-vinyl alcohol copolymers, and polyvinyl methyl ether; polyethylene glycol, polypropylene glycol, and polyoxyethylene-polyoxypropylene copolymers; amphoteric methacrylic acid ester copolymers, such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers, and (alkyl acrylate/diacetone acrylamide) copolymers AMP; partially saponified polyvinyl acetate and maleic acid copolymers; vinylpyrrolidone-dialkylaminoalkyl methacrylate copolymers; acrylic resin alkanolamine; polyester and water-dispersible polyester; polyacrylamide; copolymers of polyacrylic esters such as polyethyl acrylate, carboxy vinyl polymers, polyacrylic acid and salts thereof, such as sodium salt, and acrylic acid-methacrylic acid ester copolymers; acrylic acid-alkyl methacrylate copolymers; cationized celluloses such as polyquaternium-10, diallyldimethylammonium chloride-acrylamide copolymers, such as polyquaternium-7, acrylic acid-diallyldimethylammonium chloride copolymers, such as polyquaternium-22, acrylic acid-diallyldimethylammonium chloride-acrylamide copolymers, such as polyquaternium-39, acrylic acid-cationized methacrylic acid ester copolymers, acrylic acid-cationized methacrylic acid amide copolymers, acrylic acid-methyl acrylate-methacrylamidopropyltrimethylammonium chloride copolymers, such as polyquaternium-47, and methacrylic acid chloride choline ester polymers; cationized polysaccharides, such as cationized oligosaccharides, cationized dextran, and guar hydroxypropyltrimonium chloride; polyethyleneimine; cationic polymers; copolymers of 2-methacryloyloxyethyl phosphorylcholine and butyl methacrylate, such as polyquaternium-51; polymer emulsions, such as acrylic resin emulsions, polyethyl acrylate emulsions, polyacrylalkyl ester emulsions, polyvinyl acetate resin emulsions, natural rubber latex, and synthetic latex; nitrocellulose; polyurethanes and various copolymers thereof; various silicones; various silicone copolymers, such as acrylic-silicone graft copolymers; various fluorine polymers; 12-hydroxystearic acid and salts thereof; dextrin fatty acid esters, such as dextrin palmitate and dextrin myristate; and silicic anhydride, fumed silica (silicic anhydride ultrafine particles), magnesium aluminum silicate, magnesium sodium silicate, metallic soaps, dialkyl phosphate metal salts, bentonite, hectorite, organic-modified clay minerals, sucrose fatty acid esters, and fructooligosaccharide fatty acid esters. Among the aforementioned examples, preferred are cellulose and derivatives thereof, alginic acid and salts thereof, polyvinyl alcohol, hyaluronic acid and salts thereof, or collagen.

Preferred examples of solvents include lower alcohols, such as ethanol, 2-propanol (isopropyl alcohol), butanol, and isobutyl alcohol; glycols, such as propylene glycol, diethylene glycol, dipropylene glycol, and isopentyldiol; glycol ethers, such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters, such as ethylene glycol monoethyl ether acetate, diethylene glycol monoethyl ether acetate, and propylene glycol monoethyl ether acetate; glycol esters, such as diethoxyethyl succinate and ethylene glycol disuccinate; benzyl alcohol, benzyloxyethanol, propylene carbonate, dialkyl carbonate, acetone, ethyl acetate, and N-methylpyrrolidone; and toluene.

Preferred examples of antioxidants include tocopherol (vitamin E) and tocopherol derivatives, such as tocopherol acetate; BHT and BHA; gallic acid derivatives, such as propyl gallate; vitamin C (ascorbic acid) and/or derivatives thereof; erythorbic acid and derivatives thereof; sulfites, such as sodium sulfite; hydrogen sulfites, such as sodium hydrogen sulfite; thiosulfates, such as sodium thiosulfate; metabisulfites; thiotaurine and hypotaurine; and thioglycerol, thiourea, thioglycolic acid, and cysteine hydrochloride.

Preferred examples of reducing agents include thioglycolic acid, cysteine, and cysteamine.

Preferred examples of oxidizers include hydrogen peroxide solution, ammonium persulfate, sodium bromate, and percarbonic acid.

Preferred examples of preservatives, antimicrobial agents, and antiseptics include hydroxybenzoic acids and salts or esters thereof, such as methylparaben, ethylparaben, propylparaben, and butylparaben; salicylic acid; sodium benzoate; phenoxyethanol; isothiazolinone derivatives, such as methylchloroisothiazolinone and methylisothiazolinone; imidazolinium urea; dehydroacetic acid and salts thereof; phenols; halogenated bisphenols, such as triclosan, acid amides thereof, and quaternary ammonium salts thereof; trichlorocarbanide, zinc pyrithione, benzalkonium chloride, benzethonium chloride, sorbic acid, chlorhexidine, chlorhexidine gluconate, halocarban, hexachlorophene, and hinokitiol; other phenols, such as phenol, isopropylphenol, cresol, thymol, parachlorophenol, phenylphenol, and sodium phenylphenate; phenyl ethyl alcohol, photosensitizers, antibacterial zeolite, and silver ions.

Preferred examples of chelating agents include edetates (ethylenediamine tetraacetates), such as EDTA, EDTA 2Na, EDTA 3Na, and EDTA 4Na; hydroxyethylethylenediamine triacetates, such as HEDTA 3Na; pentetates (diethylenetriamine pentaacetate); phytic acid; phosphonic acids, such as etidronic acid, and salts thereof, such as sodium salt; polyamino acids, such as polyaspartic acid and polyglutamic acid; sodium polyphosphate, sodium metaphosphate, and phosphoric acid; and sodium citrate, citric acid, alanine, dihydroxyethylglycine, gluconic acid, ascorbic acid, succinic acid, and tartaric acid.

Preferred examples of pH adjusters, acids, and alkalis include citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, aqueous ammonia, guanidine carbonate, and ammonium carbonate.

Preferred examples of powders include inorganic powders having various sizes and shapes, such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, zeolite, barium sulfate, baked calcium sulfate, calcium phosphate, fluorapatite, hydroxyapatite, ceramic powder, bentonite, smectite, clay, mud, metallic soaps (e.g., zinc myristate, calcium palmitate, and aluminum stearate), calcium carbonate, red iron oxide, yellow iron oxide, black iron oxide, ultramarine, Prussian blue, carbon black, titanium oxide, titanium oxide fine particles and titanium oxide ultrafine particles, zinc oxide, zinc oxide fine particles and zinc oxide ultrafine particles, alumina, silica, fumed silica (silicic anhydride ultrafine particles), titanated mica, argentine, boron nitride, photochromic pigments, synthetic fluorophlogopite, particulate composite powders, gold, and aluminum; inorganic powders, such as hydrophobic or hydrophilic powders prepared by treating the aforementioned powders with various surface-treating agents such as silicones (e.g., hydrogen silicone and cyclic hydrogen silicone) or other silanes or titanium coupling agents; organic powders having various sizes and shapes, such as starch, cellulose, nylon powder, polyethylene powder, polymethyl methacrylate powder, polystyrene powder, styrene-acrylic acid copolymer resin powder, polyester powder, benzoguanamine resin powder, polyethylene terephthalate-polymethyl methacrylate laminated powder, polyethylene terephthalate-aluminum-epoxy laminated powder, urethane powder, silicone powder, and Teflon (registered trademark) powder; surface-treated organic powders; and organic-inorganic composite powders.

Preferred examples of inorganic salts include sodium chloride-containing salts, such as common salt, regular salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; and sodium phosphates, such as mono-, di-, and tri-sodium phosphates, potassium phosphates, calcium phosphates, and magnesium phosphates.

Preferred examples of ultraviolet absorbers include benzoic acid ultraviolet absorbers, such as p-aminobenzoic acid, p-aminobenzoic acid monoglycerin ester, N,N-dipropoxy-p-aminobenzoic acid ethyl ester, N,N-diethoxy-p-aminobenzoic acid ethyl ester, N,N-dimethyl-p-aminobenzoic acid ethyl ester, N,N-dimethyl-p-aminobenzoic acid butyl ester, and N,N-dimethyl-p-aminobenzoic acid methyl ester; anthranilic acid ultraviolet absorbers, such as homomenthyl-N-acetylanthranilate; salicylic acid ultraviolet absorbers, such as salicylic acid and sodium salt thereof, amyl salicylate, menthyl salicylate, homomenthyl salicylate, octyl salicylate, phenyl salicylate, benzyl salicylate, and p-isopropanolphenyl salicylate; cinnamic acid ultraviolet absorbers, such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate (octyl p-methoxycinnamate), 2-ethoxyethyl-p-methoxycinnamate (cinoxate), cyclohexyl-p-methoxycinnamate, ethyl-α-cyano-β-phenylcinnamate, 2-ethylhexyl-α-cyano-β-phenylcinnamate (octocrylene), glyceryl mono-2-ethylhexanoyl-di-p-methoxycinnamate, ferulic acid, and derivatives thereof; benzophenone ultraviolet absorbers, such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,l-camphor and 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives, such as 4-t-butylmethoxydibenzoylmethane; octyl triazone; urocanic acid and urocanic acid derivatives, such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives, such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of whitening agents include hydroquinone glycosides, such as arbutin and α-arbutin, and esters thereof; ascorbic acid, and ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbic acid alkyl ethers, such as ascorbic acid ethyl ether, ascorbic acid glucosides, such as ascorbic acid 2-glucoside and fatty acid esters thereof, ascorbyl sulfate, and tocopheryl ascorbyl phosphate; kojic acid, ellagic acid, tranexamic acid and derivatives thereof; ferulic acid and derivatives thereof; placenta extract, glutathione, oryzanol, butylresorecinol, and plant extracts, such as oil-soluble chamomilla extract, oil-soluble licorice extract, Tamarix chinensis extract, and Saxifraga stolonifera extract.

Preferred examples of vitamins and derivatives thereof include forms of vitamin A, such as retinol, retinol acetate, and retinol palmitate; forms of vitamin B, such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acid products, nicotinic acid products, such as nicotinamide and benzyl nicotinate, and choline products; forms of vitamin C, such as ascorbic acid and salts thereof, such as sodium salt; vitamin D; forms of vitamin E, such as α-, β-, γ-, and δ-tocopherols; other vitamins, such as pantothenic acid and biotin; ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbyl stearate, ascorbyl palmitate, and ascorbyl dipalmitate, ascorbic acid alkyl ethers, such as ascorbic acid ethyl ether, ascorbic acid glucosides, such as ascorbic acid-2-glucoside and fatty acid esters thereof, and tocopheryl ascorbyl phosphate; vitamin derivatives, for example, tocopherol derivatives, such as tocopherol nicotinate, tocopherol acetate, tocopherol linoleate, tocopherol ferulate, and tocopherol phosphate, tocotrienol, and other various vitamin derivatives.

Preferred examples of hair growth-promoting agents, blood circulation promoters, and stimulants include plant extracts and tinctures, such as swertia herb extract, capsicum tincture, ginger tincture, ginger extract, and cantharis tincture; capsaicin, nonylic acid vanillylamide, zingerone, ichthammol, tannic acid, borneol, cyclandelate, cinnarizine, tolazoline, acetylcholine, verapamil, cepharanthine, γ-oryzanol, vitamin E and derivatives thereof, such as tocopherol nicotinate and tocopherol acetate, γ-oryzanol, nicotinic acid and derivatives thereof, such as nicotinamide, benzyl nicotinate, inositol hexanicotinate, and nicotinic alcohol, allantoin, photosensitizer 301, photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone, and prednisone. Preferred examples of other medicinal agents, such as anti-wrinkle agents, anti-aging agents, firming agents, cooling agents, warming agents, wound-healing promoters, abirritants, analgesics, and cell activators include retinol products, retinoic acid products, and tocopheryl retinoate; lactic acid, glycolic acid, gluconic acid, fruit acid, salicylic acid, and derivatives thereof, such as glycosides and esters, and α- or β-hydroxy acids and derivatives thereof, such as hydroxycapric acid, long-chain α-hydroxy fatty acids, and long-chain α-hydroxy fatty acid cholesteryl esters; γ-aminobutyric acid and γ-amino-β-hydroxybutyric acid; carnitine; carnocin; creatine; ceramides and sphingosines; caffeine, xanthine, and derivatives thereof; antioxidants and active oxygen scavengers, such as coenzyme Q10, carotene, lycopene, astaxanthin, lutein, α-lipoic acid, platinum nanocolloid, and fullerenes; catechins; flavones, such as quercetin; isoflavones; gallic acid and sugar ester derivatives thereof; polyphenols, such as tannin, sesamin, proanthocyanidin, chlorogenic acid, and apple polyphenols; rutin and derivatives thereof, such as glycosides; hesperidin and derivatives thereof, such as glycosides; lignan glycosides; licorice extract-related substances, such as glabridin, glabrene, liquiritin, and isoliquiritin; lactoferrin; shogaol and gingerol; perfume substances, such as menthol and cedrol, and derivatives thereof; capsaicin, vanillin, and derivatives thereof; insect repellents, such as diethyltoluamide; and complexes of physiologically active substances and cyclodextrins.

Preferred examples of plant, animal, and microbial extracts include iris extract, Angelica keiskei extract, Thujopsis dolabrata extract, asparagus extract, avocado extract, Hydrangea serrata leaf extract, almond extract, Althea officinalis root extract, Arnica montana extract, aloe extract, apricot extract, apricot kernel extract, ginkgo extract, Artemisia capillaris flower extract, fennel fruit extract, turmeric root extract, oolong tea extract, uva-ursi extract, rose fruit extract, Echinacea angustifolia leaf extract, Isodonis japonicus extract, Scutellaria root extract, Phellodendron bark extract, Coptis rhizome extract, barley extract, Panax ginseng extract, Hypericum perforatum extract, Lamium album extract, Ononis spinosa extract, Nasturtium officinale extract, orange extract, dried sea water residues, seaweed extract, Japanese persimmon leaf extract, Pyracantha fortuneana fruit extract, hydrolyzed elastin, hydrolyzed wheat powder, hydrolyzed silk, Pueraria root extract, Chamomilla recutita extract, oil-soluble Chamomilla recutita extract, carrot extract, Artemisia capillaris extract, Avena fatua extract, Hibiscus sabdariffa extract, licorice extract, oil-soluble licorice extract, kiwi fruit extract, kiou extract, Auricularia auricula-judae extract, Cinchona bark extract, cucumber extract, Paulownia tomentosa leaf extract, guanosine, guava extract, Sophora root extract, Gardenia jasminoides extract, Sasa veitchii extract, Sophora flavescens extract, walnut extract, chestnut extract, grapefruit extract, Clematis vitalba extract, black rice extract, black sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, Asiasarum root extract, Bupleurum root extract, umbilical cord extract, saffron extract, salvia extract, Saponaria officinalis extract, bamboo grass extract, Crataegus cuneata fruit extract, Bombyx mori excrementum extract, Zanthoxylum piperitum peel extract, shiitake mushroom extract, Rehmannia glutinosa root extract, Lithospermum root extract, Perilla frutescens extract, Tilia japonica extract, Filipendula multijuga extract, jatoba extract, peony root extract, ginger extract, Acorus calamus root extract, Betula alba extract, Tremella fuciformis extract, Equisetum arvense extract, stevia extract, stevia fermentation product, Tamarix chinensis extract, Hedera helix extract, Crataegus oxycantha extract, Sambucus nigra extract, Achillea millefolium extract, Mentha piperita extract, sage extract, mallow extract, Cnidium rhizome extract, swertia herb extract, mulberry bark extract, rhubarb extract, soybean extract, jujubi extract, thyme extract, dandelion extract, lichens extract, tea extract, clove extract, Imperata cylindrica extract, Citrus unshiu peel extract, tea tree oil, Rubus suavissimus extract, capsicum extract, Angelica acutiloba root extract, Calendula officinalis extract, peach kernel extract, bitter orange peel extract, Houttuynia cordata extract, tomato extract, natto extract, carrot extract, garlic extract, Rosa canina fruit extract, hibiscus extract, Ophiopogon tuber extract, lotus extract, parsley extract, birch extract, honey, Hamamelis virginiana extract, Parietaria officinalis extract, Rabdosia japonica extract, bisabolol, cypress extract, Bifidobacterium extract, Eriobotiya japonica extract, Tussilago farfara extract, Japanese butterbur flower-bud extract, Poria cocos sclerotium extract, butcher's broom extract, grape extract, grape seed extract, propolis, Luffa cylindrica extract, safflower extract, peppermint extract, Tilia miqueliaria extract, Paeonia suffruticosa extract, hop extract, Rosa rugosa extract, pine extract, Aesculus hippocastanum extract, Lysichiton camtschatcense extract, Sapindus mukurossi extract, Melissa officinalis extract, Nemacystus decipiens extract, peach extract, cornflower extract, Eucalyptus globulus extract, Saxifraga stolonifera extract, Citrus junos extract, lily extract, coix seed extract, Artemisia princeps extract, lavender extract, green tea extract, egg shell membrane extract, apple extract, rooibos tea extract, Litchi chinensis extract, lettuce extract, lemon extract, Forsythia fruit extract, Astragalus sinicus extract, rose extract, rosemary extract, Anthemis nobilis extract, royal jelly extract, and Sanguisorba officinalis extract.

Examples of antipruritics include diphenhydramine hydrochloride, chlorpheniramine maleate, camphor, and substance-P inhibitors.

Examples of exfoliates/keratolytic agents include salicylic acid, sulfur, resorcin, selenium sulfide, and pyridoxine.

Examples of antiperspirants include aluminum chlorohydrate, aluminum chloride, zinc oxide, and zinc p-phenolsulfonate.

Examples of algefacients include menthol and methyl salicylate.

Examples of astringents include citric acid, tartaric acid, lactic acid, aluminum potassium sulfate, and tannic acid.

Examples of enzymes include superoxide dismutase, catalase, lysozyme chloride, lipase, papain, pancreatin, and protease.

Preferred examples of nucleic acids include ribonucleic acids and salts thereof, deoxyribonucleic acids and salts thereof, and adenosine triphosphate disodium.

Preferred examples of perfumes include synthetic and natural perfumes and various compound perfumes, such as acetyl cedrene, amylcinnamaldehyde, allylamyl glycolate, β-ionone, Iso E Super, isobutylquinoline, iris oil, irone, indole, ylang ylang oil, undecanal, undecenal, γ-undecalactone, estragole, eugenol, oakmoss, opoponax resinoid, orange oil, eugenol, aurantiol, galaxolide, carvacrol, L-carvone, camphor, canon, carrot seed oil, clove oil, methyl cinnamate, geraniol, geranyl nitrile, isobornyl acetate, geranyl acetate, dimethylbenzylcarbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydrojasmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinen, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexylcinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methylnonylacetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, and various essential oils.

Preferred examples of colors, coloring agents, and dyes include certified colors, such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green. No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407, and Yellow No. 5; other acid dyes, such as Acid Red 14; basic dyes, such as Arianor Sienna Brown, Arianor Madder Red, Arianor Steel Blue, and Arianor Straw Yellow; nitro dyes, such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2, and Basic Blue 26; disperse dyes; natural colors and dyes, for example, anthraquinones, such as astaxanthin and alizarin, naphthoquinones, such as anthocyanidin, β-carotene, catenal, capsanthin, chalcone, carthamin, quercetin, crocin, chlorophyll, curcumin, cochineal, and shikonin, bixin, flavones, betacyanidine, henna, hemoglobin, lycopene, riboflavin, and rutin; oxidation dye intermediates and couplers, such as p-phenylenediamine, toluene-2,5-diamine, o-, m-, or p-aminophenol, m-phenylenediamine, 5-amino-2-methylphenol, resorcin, 1-naphthol, and 2,6-diaminopyridine, and salts thereof; autoxidation dyes, such as indoline; and dihydroxyacetone.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphedramine hydrochloride, and chlorpheniramine maleate; and plant extracts, such as peach leaf extract and Artemisia princeps leaf extract.

Preferred examples of anti-asthmatic agents, anti-chronic obstructive pulmonary disease agents, anti-allergic agents, and immunomodulators include aminophylline, theophyllines, steroids (e.g., fluticasone and beclomethasone), leukotriene antagonists, thromboxane inhibitors, Intal, β2 agonists (e.g., formoterol, salmeterol, albuterol, tulobuterol, clenbuterol, and epinephrine), tiotropium, ipratropium, dextromethorphan, dimemorfan, bromhexine, tranilast, ketotifen, azelastine, cetirizine, chlorpheniramine, mequitazine, tacrolimus, ciclosporin, sirolimus, methotrexate, cytokine modulators, interferon, omalizumab, and protein/antibody preparations.

Preferred examples of anti-infective agents and antifungal agents include oseltamivir, zanamivir, and itraconazole. The stick-type solid base material for external application to skin may contain, besides the aforementioned ingredients, known cosmetic ingredients, known pharmaceutical ingredients, and known food ingredients, such as ingredients described in Japanese Standards of Cosmetic Ingredients, Japanese Cosmetic Ingredients Codex, List of Cosmetics Ingredients Japanese Labeling Names issued by the Japan Cosmetic Industry Association, INCI dictionary (The International Cosmetic Ingredient Dictionary and Handbook), Japanese Standards of Quasi-drug Ingredients, Japanese Pharmacopoeia, Japanese Pharmaceutical Excipients, Japan's Specifications and Standards for Food Additives, and other standards, and ingredients described in Japanese and foreign patent publications and patent application publications (including Japanese translations of PCT international application publications and re-publications of PCT international publications) classified as International Patent Classification IPC classes A61K7 and A61K8, in known combinations and in known proportions and amounts.

### [Method for Producing Stick-Type Solid Base Material for External Application to Skin]

The stick-type solid base material for external application to skin of the present invention can be produced through the following procedure: a lipidic peptide compound composed of at least one of compounds of Formulae (1) to (3) or pharmaceutically usable salts thereof is mixed with a surfactant and water, a 1,2-alkanediol or a 1,3-alkanediol, a fatty acid, an oily base, a higher alcohol, and optionally with a pigment and other additives under heating with stirring; and then the resultant mixture is allowed to stand still and cool.

For example, the stick-type solid base material for external application to skin of the present invention is produced by the following steps of:
a) mixing the lipidic peptide compound with a surfactant and water, and heating the mixture to thereby prepare a solution or a dispersion;
b) adding the solution or the dispersion to water, and heating the mixture at a temperature equal to or higher than room temperature and lower than 100°C; and
c) cooling the mixture with stirring to a temperature lower than the temperature in the aforementioned heating step, and then allowing the mixture to stand still and cool, to thereby form a gel solid (stick-type solid base material for external application to skin).

The 1,2-alkanediol or the 1,3-alkanediol, the fatty acid, the oily base, the higher alcohol, and the pigment and other additives may be added in the step of preparing the solution or the dispersion; i.e., in step a), or may be previously added to the water to which the solution or the dispersion is to be added in step b).

The amount of water is preferably 20% by mass or more and less than 95% by mass relative to the total mass of the resultant stick-type solid base material for external application to skin.

The amount of water is preferably 30% by mass or more and less than 80% by mass relative to the total mass of the prepared solution or dispersion.

In steps a) and b), the heating temperature is preferably 50°C to 90°C, more preferably 60°C to 90°C, for example, 80°C. Preferably, the mixture is stirred under heating. The heating and stirring time in each of these steps may vary depending on the types and amounts of the lipidic peptide compound and other ingredients (e.g., surfactant) used in the steps. Generally, these ingredients can be dissolved or dispersed within about 5 minutes to 50 minutes.

Steps a) and b) are followed by cooling of the mixture with stirring until the liquid temperature becomes lower than the temperature in step b) (step c)). The cooling temperature is, for example, room temperature to 80°C, room temperature to 60°C, or room temperature to about 40°C.

The stick-type solid base material for external application to skin of the present invention may also be produced by preparation of an aqueous composition (i.e., premix) and then appropriate addition of other ingredients to the premix.

For example, the stick-type solid base material for external application to skin is produced by a method involving preparation of a premix as follows.
1) Step of preparing premix
   1-1) Step of heating a mixture system containing a surfactant and water, and a lipidic peptide compound composed of at least one of compounds of Formulae (1) to (3) or pharmaceutically usable salts thereof to a temperature equal to or higher than room temperature and lower than 100°C.
   1-2) Step of appropriately adding a 1,2-alkanediol or a 1,3-alkanediol, a fatty acid, and other additives to the heated mixture system.
   1-3) Step of cooling the resultant solution or dispersion to thereby prepare an aqueous composition (premix).
2) Step of producing stick-type solid base material for external application to skin from premix
   2-1) Mixing step in which the aqueous composition (premix) heated to a temperature equal to or higher than room temperature and lower than 100°C is added to and mixed with an aqueous phase heated to a temperature equal to or higher than room temperature and lower than 100°C, or an aqueous phase heated to a temperature equal to or higher than room temperature and lower than 100°C is added to and mixed with the aqueous composition (premix) heated to a temperature equal to or higher than room temperature and lower than 100°C.
   2-2) Cooling step in which the mixture prepared in the mixing step is cooled to thereby form a stick-type solid base material (gel) for external application to skin.

In step 2-1), the aqueous phase contains water, and may further contain an oily base, a higher alcohol, a pigment, a 1,2-alkanediol or a 1,3-alkanediol, a fatty acid, and other additives. In step 2-1), the aqueous composition (premix) may be mixed with a pigment and other additives, and then the mixture may be mixed with the aqueous phase.

In the aforementioned mixing step, a drug solution may be further added to the mixture to thereby produce a drug-containing stick-type solid base material (preparation) for external application to skin.

In steps 1-1) and 1-2), the heating temperature of the mixture system (and the aqueous composition) is preferably 50°C to 90°C, more preferably 60°C to 90°C, for example, 70°C or 80°C. These steps are preferably performed with stirring. The heating (stirring) time in each of these steps may vary depending on the types and amounts of the lipidic peptide compound and other ingredients (e.g., surfactant) contained in the aqueous composition. Generally, the heating (stirring) time is about 5 minutes to 50 minutes. The aqueous composition is homogeneously dissolved through these steps.

In step 1-3), the cooling temperature is, for example, room temperature to 80°C, room temperature to 60°C, or room temperature to about 40°C. In this step, the aqueous composition is preferably cooled with stirring. More preferably, the stirring is then stopped, and the aqueous composition is allowed to stand still.

The amount of water is preferably 30% by mass or more and less than 80% by mass relative to the total mass of the aqueous composition (premix) prepared by step 1).

In step 2-1), the heating temperature of the aqueous phase and the aqueous composition (premix) is preferably 50°C to 90°C, more preferably 60°C to 90°C, for example, 70°C or 80°C or 90°C. In particular, the aqueous phase is preferably heated with stirring, since it contains other ingredients (e.g., an oily base). The heating (stirring) of the aqueous phase is preferably performed for generally about 5 minutes to 50 minutes until the ingredients contained in the aqueous phase are homogeneously dissolved or dispersed. The heating temperature of the aqueous phase may be equal to that of the aforementioned aqueous composition (premix).

Subsequently, in step 2-2), the mixture prepared in the preceding step is cooled to thereby form a gel solid (stick-type solid base material for external application to skin). In this step, the mixture may be cooled with stirring. In the case where the mixture is cooled with stirring, preferably, the stirring is performed until, for example, the cooling temperature becomes room temperature to 80°C or room temperature to 60°C, for example, about 60°C, and then the stirring is stopped and the mixture is allowed to stand still and cool. In particular, preferably, the stirring is stopped at 50°C or lower, and the mixture is allowed to stand still and cool.

In the stick-type solid base material for external application to skin produced from the aqueous composition (premix) as described above, the amount of water is preferably 20% by mass or more and less than 95% by mass relative to the total mass of the solid base material for external application to skin.

### Examples

The present invention will next be described in detail with reference to examples and test examples, but the present invention is not limited to the following examples.

### [Synthesis Example 1: Synthesis of Lipidic Peptide (N-Palmitoyl-Gly-His)]

The lipidic peptide compound used in the examples was synthesized by the method described below.

A 500 mL four-necked flask was charged with 14.2 g (91.6 mmol) of histidine, 30.0 g (91.6 mmol) ofN-palmitoyl-Gly-methyl, and 300 g of toluene, and 35.3 g (183.2 mmol) of a 28% methanol solution of sodium methoxide serving as a base was added to the flask. The mixture was heated to 60°C in an oil bath and continuously stirred for one hour. Thereafter, the oil bath was removed, and the mixture was allowed to cool to 25°C. To the resultant solution was added 600 g of acetone for reprecipitation, and the precipitate was separated by filtration. The resultant solid was dissolved in a mixed solution of 600 g of water and 750 g of methanol. To the solution was added 30.5 ml (183.2 mmol) of 6N hydrochloric acid to thereby neutralize the solution and precipitate a solid, and the solid was filtered. Subsequently, the resultant solid was dissolved in a mixed solution of 120 g of tetrahydrofuran and 30 g of water at 60°C, and 150 g of ethyl acetate was added to the solution. The resultant mixture was cooled from 60°C to 30°C, and then the precipitated solid was filtered. The resultant solid was dissolved in a solvent mixture of 120 g of tetrahydrofuran and 60 g of acetonitrile. The solution was heated to 60°C, stirred for one hour, and then cooled, followed by filtration. The resultant solid was washed with 120 g of water, filtered, and then dried under reduced pressure, to thereby produce 26.9 g of a free form of N-palmitoyl-Gly-His (hereinafter may be also referred to simply as "Pal-GH") as white crystals (yield: 65%).

### [Example 1: Preparation of Stick-Type Solid Base Material for External Application to Skin Containing 20% by Mass Oily Base (1)]

Stick-type solid base materials for external application to skin each containing 20% by mass an oily base were produced as shown in Table 1 below.

In a Laboran screw tube vial <1> (No. 5, available from AS ONE Corporation), 0.15 g of a higher alcohol was dissolved in 2.85 g of pure water at 84°C with stirring, and the solution was mixed with 2.0 g of an oily base heated to 84°C with stirring. In Comparative Example, a higher alcohol was not used, and 2.0 g of an oily base was mixed with 3.00 g of pure water with stirring under the same heating conditions as described above.

In another Laboran screw tube vial <2> (No. 5), 5.0 g of composition [1] was weighed and stirred at 84°C. Composition [1] having the formulation shown in Table 1 was prepared as follows: Pal-GH, polyoxyethylene lauryl ether, and purified water were mixed together and heated to 70°C with stirring; 1,2-hexanediol and stearic acid were added to the mixture; the mixture was stirred under heating at 70°C; and the resultant mixture was allowed to cool to room temperature.

Composition [1] heated to 84°C was added to the Laboran screw tube vial <1> containing, for example, the oily base heated with stirring at 84°C, and the resultant mixture was stirred.

Thereafter, the mixture was cooled to 55°C with stirring, and the resultant mixture was charged into a small metallic gastight container (available from HIDAN CO., LTD.). The mixture was cooled to room temperature to thereby produce a stick-type solid base material for external application to skin.

In the aforementioned production steps, all stirring operations were performed at 200 rpm.

The ingredients used in the examples are as follows.

Higher alcohols (at least one saturated or unsaturated monohydric alcohol having a carbon atom number of 8 to 30) used were cetanol, stearyl alcohol, and behenyl alcohol. Cetanol was obtained from KOKYU ALCOHOL KOGYO CO., LTD., and stearyl alcohol and squalane oil were obtained from Tokyo Chemical Industry Co., Ltd.

Oily bases used are as follows: TRIFATC-24, purified avocado oil, squalane oil, olive oil, apricot kernel oil, kukui nut oil, grape seed oil, safflower oil, sweet almond oil, and corn germ oil (obtained from Nikko Chemicals Co., Ltd.); mineral oil, camellia oil, aqua jojoba oil, macadamia nut oil, and sunflower oil (purchased from PINOA Co., Ltd.); liquid paraffin (obtained from KANEDA Co., Ltd.); TOG (glyceryl tri-2-ethylhexanoate), IPM-R (isopropyl myristate), IPIS (isopropyl isostearate), and HAIMALATE DIS (malic acid diester) (obtained from KOKYU ALCOHOL KOGYO CO., LTD.); and KF96A-100CS (dimethicone), KF96A-500CS (dimethicone), and KF995 (cyclopentanesiloxane) (obtained from Shin-Etsu Chemical Co., Ltd.). In general, "mineral oil" is also known as "liquid paraffin," and both terms refer to a mixture of liquid hydrocarbons derived from petroleum. In the following description of the examples, the terms "mineral oil" and "liquid paraffin" are used as indicated by the suppliers.

### [Evaluation of Stability of Stick-Type Solid Base Material for External Application to Skin]

Each of the stick-type solid base materials for external application to skin produced through the aforementioned procedure was stored at 50°C. After the elapse of a predetermined period of time, the surface of the base material was observed for determining the occurrence of "sweating" to thereby evaluate the stability of the base material.

Table 1 shows the formulations of the stick-type solid base materials for external application to skin of Example and Comparative Example. Table 2 shows the results of the stick-type solid base materials for external application to skin (without incorporation of a higher alcohol) of Comparative Example (Comparative Example A) stored at 50°C for one week. Table 3, Table 4, and Table 5 show the results of the stick-type solid base materials for external application to skin (higher alcohol: cetanol, stearyl alcohol, and behenyl alcohol, respectively) of Example (Example A) stored at 50°C for one month.

FIG. 1 shows the appearances of the surfaces of the stick-type solid base materials for external application to skin (without incorporation of a higher alcohol) of Comparative Example after storage at 50°C for one week [oily base used: (a) coconut oil (TRIFAT C-24), (b) mineral oil, (c) squalane oil, and (d) liquid paraffin].

**Table 1**

| Formulation of stick-type base material (1) | | | | |
|---|---|---|---|---|
| Ingredients (g) | | | Comparative Example | Example |
| Composition [1] | Pal-GH | 10 wt% | 0.50 | 0.50 |
| | Stearic acid | 1 wt% | 0.05 | 0.05 |
| | 1,2-Hexanediol | 4 wt% | 0.20 | 0.20 |
| | Polyoxyethylene lauryl ether^{*1} | 8 wt% | 0.40 | 0.40 |
| | Purified water | 77 wt% | 3.85 | 3.85 |
| | Composition [1] in total | 100 wt% | 5.00 | 5.00 |
| Higher alcohol | | | - | 0.15 |
| Purified water | | | 3.00 | 2.85 |
| Oily base | | | 2.00 | 2.00 |
| All ingredients in total | | | 10.00 | 10.00 |

| | | | | |
|---|---|---|---|---|
| *1: NIKKOL BL-4.2, available from Nikko Chemicals Co., Ltd. | | | | |

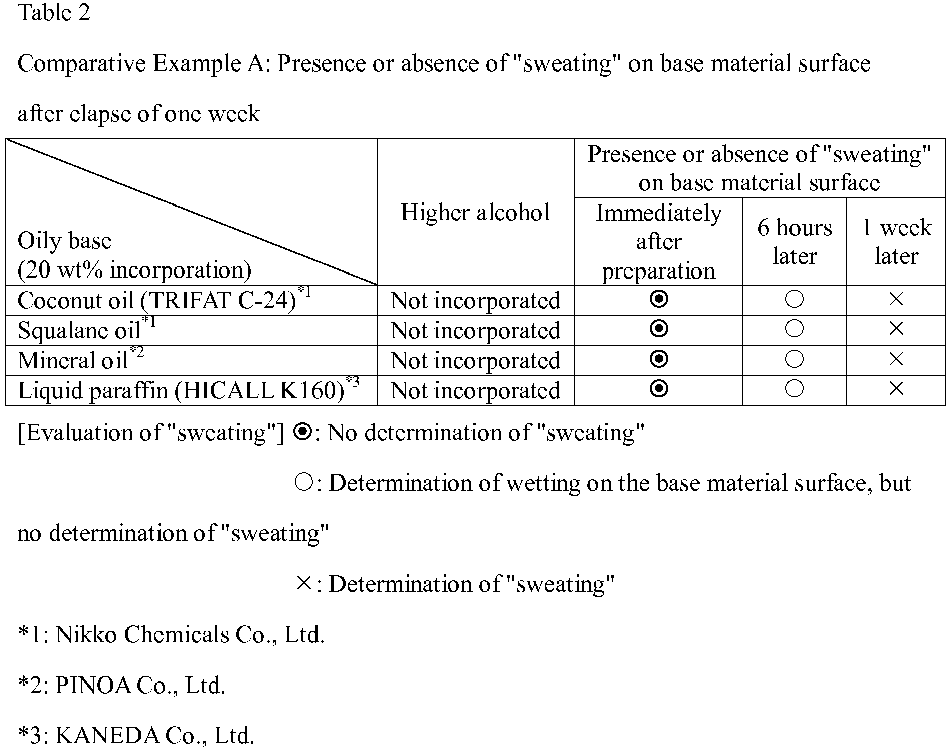

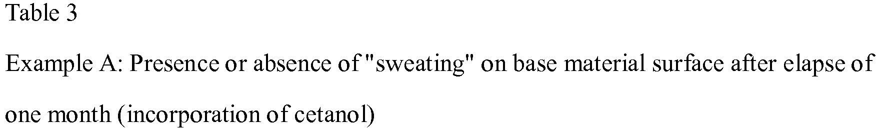

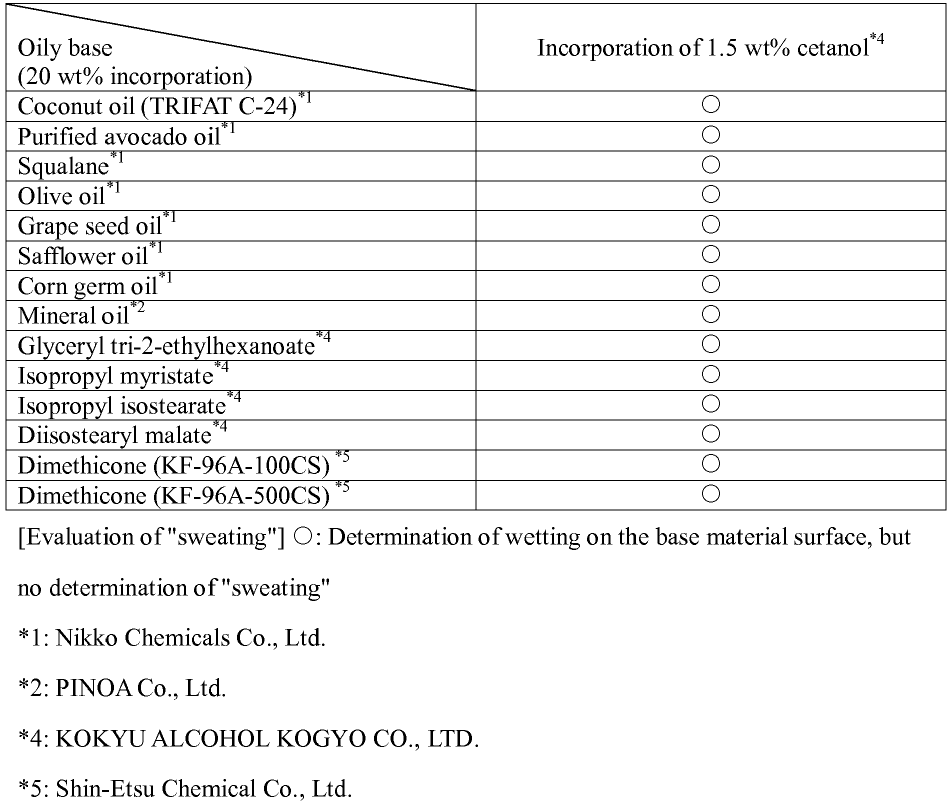

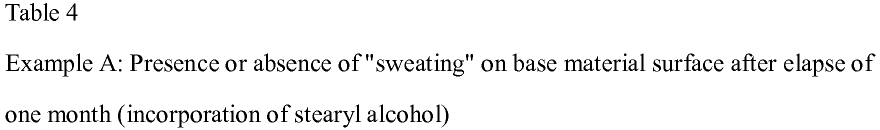

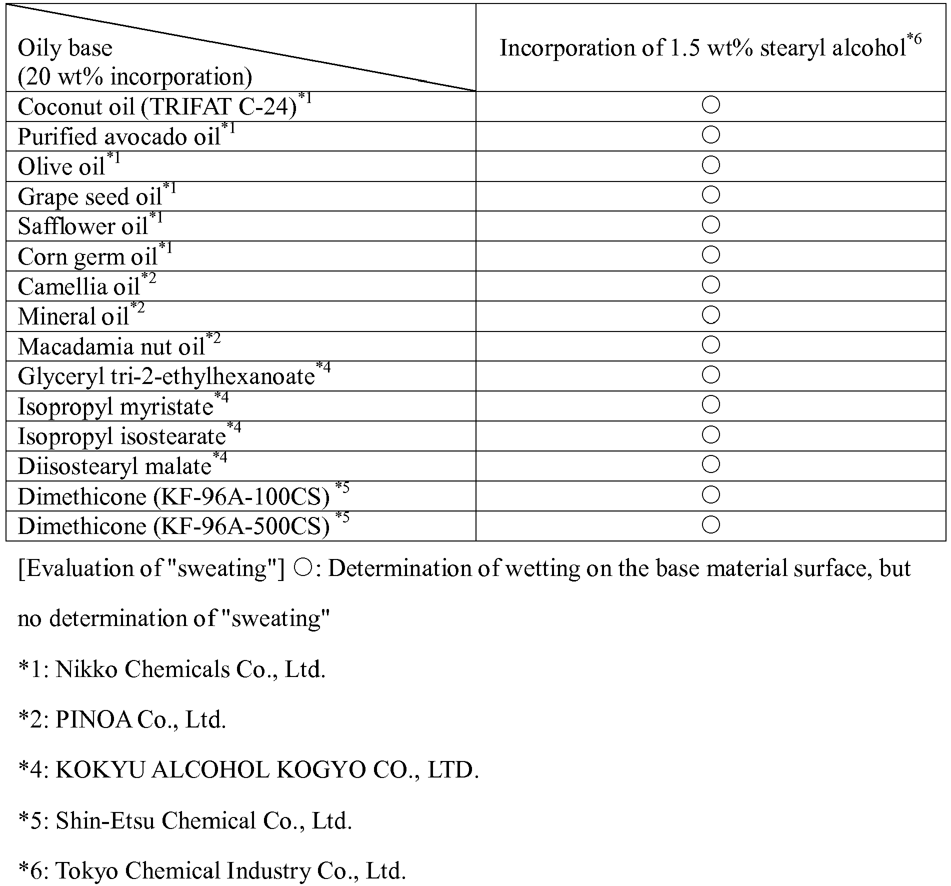

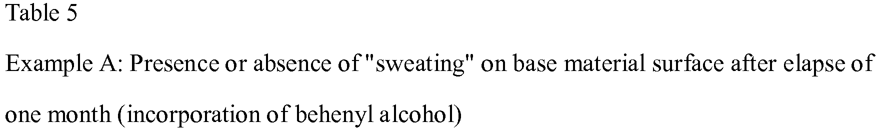

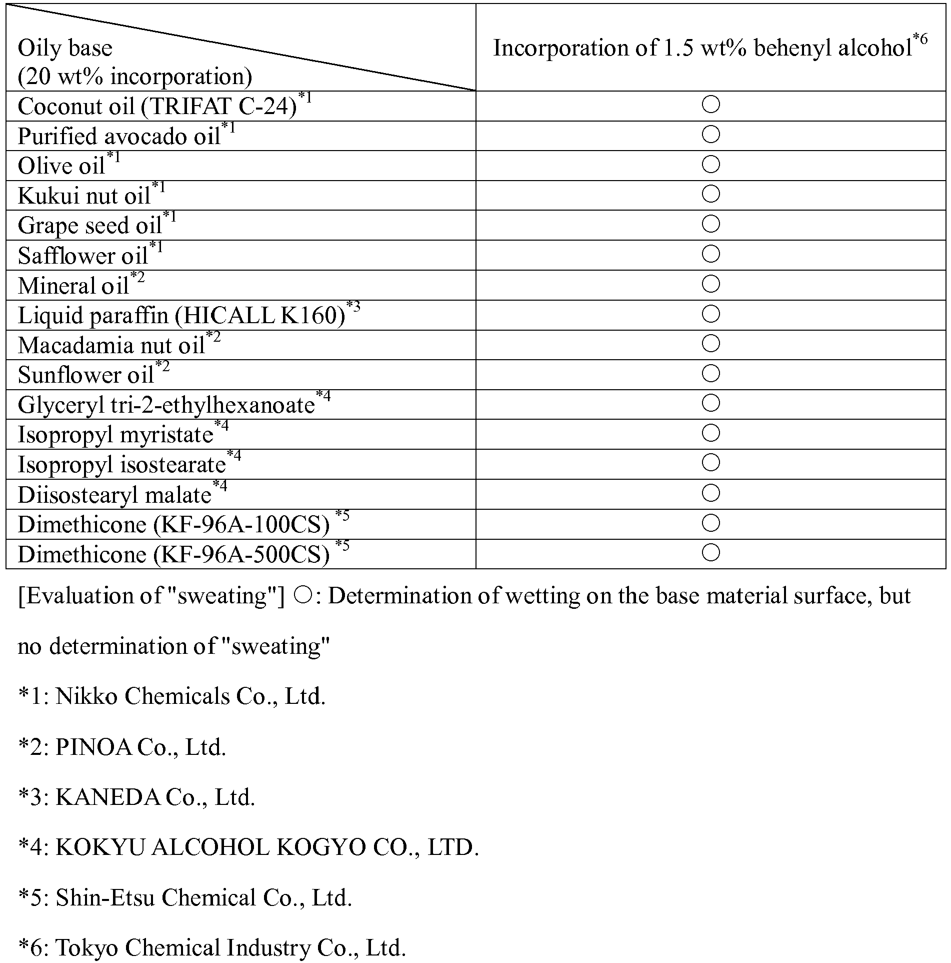

As shown in Table 2, the stick-type solid base materials for external application to skin of Comparative Example, which were prepared without incorporation of a higher alcohol (at least one saturated or unsaturated monohydric alcohol having a carbon atom number of 8 to 30), exhibited "sweating" caused by oil droplets one week later, and the base materials were found to be thinned (FIG. 1).

In contrast, as shown in Tables 3 to 5, the incorporation of a higher alcohol into a stick-type solid base material for external application to skin suppressed "sweating" of the stick-type solid base material even when it contained an oily base in an amount of 20% by mass.

These results suggest that the present invention can suppress "sweating," which has been a problem in a stick-type solid base material containing an oily base, and can also suppress thinning of the base material caused by "sweating."

### [Example 2: Preparation of Stick-Type Solid Base Material for External Application to Skin Containing 20% by Mass Oily Base (2)]

As shown in Table 6 below, stick-type solid base materials for external application to skin each containing 20% by mass an oily base were produced in the same manner and formulation as in [Example 1] (see Example in Table 1), except that a mixture of equal amounts of two oily bases was used.

Each of the thus-produced stick-type solid base materials for external application to skin was stored at 50°C. After the elapse of a predetermined period of time, the surface of the base material was observed for determining the occurrence of "sweating" to thereby evaluate the stability of the base material. The results are shown in Table 6.

FIG. 2 shows the appearances of the surfaces of the stick-type solid base materials for external application to skin (incorporation of a mixture of two oily bases) after storage at 50°C for 10 days [oily base used: (a) mineral oil + jojoba oil, (b) mineral oil + liquid paraffin, (c) squalane oil + apricot kernel oil, (d) squalane oil + kukui nut oil, (e) KF96A-500cs + KF96A-100cs, and (f) KF96A-500cs + KF995].

**Table 6**

| Formulation of stick-type base material and presence or absence of "sweating" on base material surface | | | | | | |
|---|---|---|---|---|---|---|
| Formulation | | | | Presence or absence of * "sweating"* | | |
| Composition [1] [50% by mass] | Cetanol [1.5% by mass] | Purified water [28.5% by mass] | Oily base (use of equal amounts of two ingredients) [20% by mass] | Immediately after preparation | 6 hours later | 10 days later |
| | | | Mineral oil + Liquid paraffin | ○ | ○ | ○ |
| | | | Mineral oil + Jojoba oil | ○ | ○ | ○ |
| | | | Squalane oil + Kukui nut oil | ○ | ○ | ○ |
| | | | Squalane oil + Apricot kernel oil | ○ | ○ | ○ |
| | | | KF96A-500cs + KF96A-100cs | ○ | ○ | ○ |
| | | | KF96A-500cs + KF995 | ○ | ○ | ○ |

### [Evaluation of "sweating"] ○: Determination of wetting on the base material surface, but no determination of "sweating"

As shown in Table 6, even when two oily bases were used, the stick-type solid base materials for external application to skin of the present invention were found to be able to suppress "sweating" after storage for 10 days (at 50°C), and the base materials were found not to be thinned (FIG. 2).

### [Example 3: Preparation of Stick-Type Foundation Containing 10% by Mass Pigment]

As shown in Table 7 below, 5.0 g (total) of pigment mixture (A) was stirred for 15 minutes with an automatic mortar (ANM-1000, available from NITTO KAGAKU Co., Ltd.). The compounds contained in pigment mixture (A) are as follows: sericite and talc (obtained from Dainihonkasei Co., Ltd.); and mica, titanium dioxide, silicic anhydride, yellow iron oxide, red iron oxide, and black iron oxide (purchased from Orangeflower).

In a Laboran screw tube vial <3> (No. 7, available from AS ONE Corporation), 0.5 g of cetanol was dissolved in 12.0 g of pure water at 84°C with stirring, and the solution was mixed with 10.0 g of squalane oil heated to 84°C with stirring.

In another Laboran screw tube vial <4> (No. 7, available from AS ONE Corporation), 22.5 g of composition [1] heated to 84°C was mixed with 5.0 g of pigment mixture (A) with stirring. The mixture was added to the screw tube vial <3> containing, for example, squalane oil (stirred at 84°C), and the resultant mixture was stirred. Composition [1] is the same as that described in [Example 1].

Thereafter, the mixture was cooled to 60°C with stirring, and the resultant mixture was charged into a large metallic gastight container (available from HIDAN CO., LTD.). The mixture was cooled to room temperature to thereby produce four samples of stick-type foundation.

In the aforementioned production steps, all stirring operations were performed at 200 rpm.

Cetanol was obtained from KOKYU ALCOHOL KOGYO CO., LTD., and squalane oil was obtained from Nikko Chemicals Co., Ltd.

**Table 7**

| Formulation of stick-type foundation | | | | | |
|---|---|---|---|---|---|
| Ingredients (g) | | | | | |
| Pigment mixture (A) | | Squalane oil | Cetanol | Composition [1] | Pure water |
| Talc | 2.0 g | 10.0 g | 0.5 g | 22.5 g | 12.0 g |
| Sericite | 2.0 g | | | | |
| Mica | 0.25 g | | | | |
| Titanium dioxide | 0.30 g | | | | |
| Silicic anhydride | 0.25 g | | | | |
| Yellow iron oxide | Appropriate amount^{*1} | | | | |
| Black iron oxide | Appropriate amount^{*1} | | | | |
| Red iron oxide | Appropriate amount^{*1} | | | | |

| | | | | | |
|---|---|---|---|---|---|
| *1: 0.20 g in total | | | | | |

FIG. 3 shows the appearances of the above-produced four samples of stick-type foundation. FIG. 3(a) shows the appearances immediately after production, and FIG. 3(b) shows the appearances after storage at 50°C for four weeks.

All the thus-formed stick-type foundation samples were found to have high oil and pigment dispersibility (FIG. 3(a)).

These stick-type foundation samples were found to exhibit no "sweating" on the base material surface even after storage at 50°C for four weeks, and were found not to undergo weight reduction, such as thinning (FIG. 3(b)). The results suggest that the samples exhibit excellent stability.

## Claims

1. A stick-type solid base material for external application to skin, the solid base material comprising:
a surfactant;
water;
a lipidic peptide compound comprising at least one of compounds of the following Formulae (1) to (3) or pharmaceutically usable salts of the compounds: 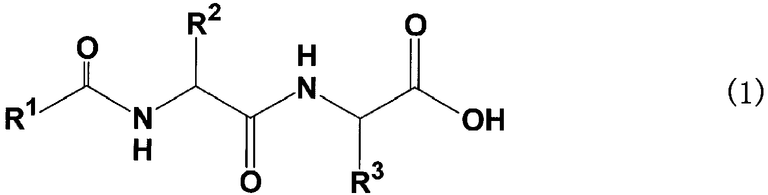 (wherein R¹ is a C₉₋₂₃ aliphatic group; R² is a hydrogen atom or a C₁₋₄ alkyl group optionally having a C₁ or C₂ branched chain; and R³ is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring), 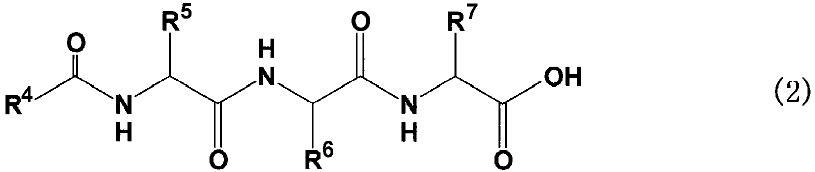 (wherein R⁴ is a C₉₋₂₃ aliphatic group; and R⁵ to R⁷ are each independently a hydrogen atom, a C₁₋₄ alkyl group optionally having a C₁ or C₂ branched chain, or a -(CH₂)ₙ-X group, and at least one of R⁵ to R⁷ is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring), and 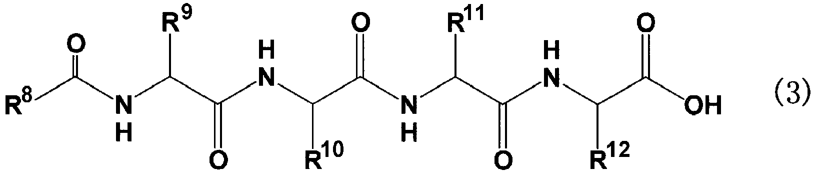 (wherein R⁸ is a C₉₋₂₃ aliphatic group; and R⁹ to R¹² are each independently a hydrogen atom, a C₁₋₄ alkyl group optionally having a C₁ or C₂ branched chain, or a -(CH₂)ₙ-X group, and at least one of R⁹ to R¹² is a -(CH₂)ₙ-X group, wherein n is a number of 1 to 4, and X is an amino group, a guanidino group, a -CONH₂ group, or a 5-membered ring or 6-membered ring optionally having one to three nitrogen atoms or a fused heterocyclic ring composed of the 5-membered ring and the 6-membered ring);
a 1,2-alkanediol or a 1,3-alkanediol;
at least one fatty acid;
an oily base; and
at least one saturated or unsaturated monohydric alcohol having a carbon atom number of 8 to 30.

2. The stick-type solid base material for external application to skin according to claim 1, wherein the monohydric alcohol is one or more compounds selected from the group consisting of cetanol, stearyl alcohol, and behenyl alcohol.

3. The stick-type solid base material for external application to skin according to claim 1 or 2, wherein the oily base is contained in an amount of 5 to 25% by mass relative to the total mass of the stick-type solid base material for external application to skin.

4. The stick-type solid base material for external application to skin according to any one of claims 1 to 3, wherein the surfactant is one or more compounds selected from the group consisting of an ethylene glycol alkyl ether, a phospholipid, a polyglycerin fatty acid ester, and a polyoxyethylene polyoxypropylene alkyl ether.

5. The stick-type solid base material for external application to skin according to any one of claims 1 to 4, wherein the fatty acid is stearic acid.

6. The stick-type solid base material for external application to skin according to any one of claims 1 to 5, wherein the solid base material further comprises a pigment.
